(19)

Europäisches
Patentamt
European
Patent Office
Office européen
des brevets

(11) **EP 3 984 630 A1**

(12) **EUROPEAN PATENT APPLICATION**

(43) Date of publication:
**20.04.2022 Bulletin 2022/16**

(21) Application number: **20201537.6**

(22) Date of filing: **13.10.2020**

(51) International Patent Classification (IPC):
**B01J 13/04** *(2006.01)*    **A61K 9/127** *(2006.01)*
**A61K 9/50** *(2006.01)*    **A61K 47/69** *(2017.01)*
**A61K 49/22** *(2006.01)*

(52) Cooperative Patent Classification (CPC):
**B01J 13/04; A61K 9/0019; A61K 9/19;**
**A61K 9/5026; A61K 9/5089; A61K 49/223**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicants:
- **Paris Sciences et Lettres**
  **75006 Paris (FR)**
- **Ecole Supérieure de Physique et de Chimie**
  **Industrielles de la Ville de Paris**
  **75005 Paris (FR)**
- **CENTRE NATIONAL DE LA RECHERCHE**
  **SCIENTIFIQUE**
  **75016 Paris (FR)**
- **Universidade Estadual de Campinas**
  **SP, CEP 13083872 Campinas (BR)**
- **Faculdades Catolicas Associacao sem fins**
  **lucrativos, mantenedora da Pontificia,**
  **Universidade Ctolica do Rio de Janeiro(PUC-Rio)**
  **Rio de Janeiro, RJ (BR)**

(72) Inventors:
- **SOYSAL, Ugur**
  **75005 PARIS (FR)**
- **NIECKELE AZEVEDO, Pedro**
  **75005 PARIS (FR)**
- **TABELING, Patrick**
  **75005 PARIS (FR)**
- **GAZIOLA DE LA TORRE, Lucimara**
  **13083-852 CAMPINAS (BR)**
- **COSTA SILVA NORONHA PESSOA, Amanda**
  **13083-852 CAMPINAS (BR)**
- **DA SILVEIRA CARVALHO, Marcio**
  **RIO DE JANEIRO-RJ (BR)**
- **MARTIN, Elian**
  **75005 PARIS (FR)**

(74) Representative: **Grosset-Fournier, Chantal**
**Catherine et al**
**Grosset-Fournier & Demachy**
**54, rue Saint-Lazare**
**75009 Paris (FR)**

(54) **FREEZE-DRIED MICROBUBBLES, THEIR USE, AND METHOD FOR PRODUCING THE SAME**

(57)    The present invention to freeze-dried monodisperse microbubbles. The invention also relates to a process for preparing said microbubbles using microfluidic techniques. The microbubbles according to the present invention show interesting properties, making them particularly useful in applications that require monodispersity of the microbubbles.

**EP 3 984 630 A1**

## Description

The present invention concerns freeze-dried microbubbles, their method of preparation, as well as their use, in particular as contrast agents.

Microbubbles find use in numerous applications such as molecular imaging, non-invasive blood pressure measurements, drug delivery, sonoporation and biosensors (Segers et. al, RSC Nanoscience and Nanotechnology, 2015, p.81-101).

For many of these applications, microbubbles having a diameter between 0.5 and 10 $\mu$m and having a low polydispersity index are desired, in particular having a polydispersity index below 10%. In clinical applications, a polydisperse set of microbubbles can present several disadvantages:

- When polydisperse microbubbles are used as contrast agents, only a fraction of the polydisperse microbubbles can resonate due to the fact that current echographs work at a narrow range of frequencies. This leads to serious limitations in the image quality.
- High polydispersity results in an impossibility to extract quantitative information, limiting their use in certain applications of medical significance such as the measurement of local pressure in a blood vessel.

In addition, since these microbubbles are susceptible to be used in a clinical environment, they should be physiologically inert, and exempt of toxic constituents, such as residual solvents. Furthermore, because the microbubbles might not be used immediately after production, they should be stored in a convenient and sufficiently stable form, preferably as a freeze-dried powder.

In this respect, the microbubbles that exist today, in particular commercialized microbubbles, do not meet the above-mentioned standards. Indeed, commercialized and clinically approved microbubbles are polydisperse and are sold in a freeze-dried form, while some other today's bubbles either contain solvents toxic to the patient, or are sold in a non-freeze-dried form.

Freeze-dried mono-disperse microbubbles are difficult, if not impossible to obtain, because the freeze-drying procedure leads to loss of monodispersity, leading to a final product that is polydisperse.

Thus, one aim of the present invention is to provide freeze-dried monodisperse microbubbles. Another aim of the present invention is to provide a process for the preparation of monodisperse freeze-dried microbubbles.

Yet another aim of the present invention is a process of resuspension of freeze-dried monodisperse microbubbles, in particular using microfluidic techniques.

Yet another aim of the present invention is to provide a suspension of monodisperse freeze-dried microbubbles.

A further aim of the present invention is the use of freeze-dried monodisperse microbubbles, in particular as contrast agents, due to their excellent echogenic properties.

Yet another aim of the present invention is the provision of a microfluidic chip comprising freeze-dried monodisperse microbubbles.

Thus, **a first object** of the present invention is freeze-dried monodisperse microbubbles, wherein:

- the microbubbles comprise a shell and a core,
- the microbubbles have a size inferior to 50 $\mu$m, in particular of from 200 nm to 50 $\mu$m, more in particular of from 1 to 50 $\mu$m, even more in particular of from 1 to 5 $\mu$m,
- the microbubbles have a polydispersity index below 10%, preferably below 6%, more in particular below 5%,

wherein the microbubbles are physiologically inert,

wherein the microbubbles are adsorbed on a hydrophilic surface in the form of at least one monolayer.

The inventors have surprisingly found that arranging monodisperse microbubbles on a hydrophilic surface, in the form of at least one monolayer, allows for subsequent freeze-drying of the microbubbles, and further resuspension, without the loss of the monodispersity and without substantial change in the value of the polydispersity index.

Without being bound to theory, when the microbubbles are arranged in a monolayer, the interactions, such as electrostatic interaction, between the microbubbles are limited as compared to the interactions between microbubbles in the form of a bulk powder, not arranged in a monolayer. These reduced interactions stabilize the microbubbles, making them less sensitive towards the potentially destructive action of freeze-drying.

For the sake of comparison, when the microbubbles are arranged in a multilayer, instead of a monolayer, the monodispersity is degraded, in particular by the phenomena of coalescence and Ostwald ripening, even before freeze-drying, even when the microbubbles are immediately transferred into the freezer.

This innovative approach of providing at least one monolayer of microbubbles, which according to the inventors has no precedent, allows access to monodisperse freeze-fried microbubbles as well as monodisperse resuspended microbubbles hereafter designated by "a suspension of microbubbles".

By *"monodisperse"* is meant that for a plurality of microbubbles (e.g. at least 100, more preferably at least 1000) the microbubbles have a polydispersity index, or coefficient of variation (CV) of their diameters of less than 10%.

The *"polydispersity index"*, or PDI, represents a measure of the size distribution of the microbubbles. The polydispersity index according to the present invention is calculated according to **Formula 1.**

$$PDI = \frac{100 \sqrt{\langle d^2\rangle - \langle d\rangle^2}}{\langle d\rangle} \qquad \textbf{Formula 1}$$

in which

    d represents the diameter of the individual microbubbles as measured by optical microscopy.
    <d> represents the arithmetic average of the measured diameters for a population of microbubbles.

With the expression *"below 10%"* relating to the polydispersity index, should also be understood the following values: below 9%, below 8%, below 7%, below 6%, below 5%, below 4%, below 3%, and below 2%, in particular the following ranges: from 1 to 10%, 1 to 9%, 1 to 8%, 1 to 7%, 1 to 6%, 1 to 5%, 1 to 4%, 1 to 3%, 1 to 2%, 2 to 9%, 3 to 8%, 4 to 7%, 5 to 6%, 2 to 10%, 3 to 10%, 4 to 10%, 5 to 10%, 6 to 10%, 7 to 10%, 8 to 10%, and 9 to 10%.

The expression *"microbubble size"* according to the invention is defined as the diameter of the microbubbles as measured by optical microscopy. The diameter is considered starting from the center of the bubble and tracing a straight line to both opposite sides of the shell of the microbubbles. The diameter corresponds to the distance between the extremities of this line, that is to say, the distance between the two opposite points on the interior side of the shell. The bubble size is determined as the arithmetic average of the bubble sizes as measured for a minimum of 100 microbubbles.

With the expression *"200 nm to 50 $\mu$m"* relating to the bubble size, should also be understood the following ranges: 200 to 500 nm, 200 to 750 nm, 200 nm to 1 $\mu$m, 200 nm to 5 $\mu$m, 200 nm to 10 $\mu$m, 200 nm to 20 $\mu$m, 200 nm to 30 $\mu$m, 200 nm to 40 $\mu$m, 500 nm to 50 $\mu$m, 750 nm to 50 $\mu$m, 1 to 50 $\mu$m, 5 to 50 $\mu$m, 10 to 50 $\mu$m, 20 to 50 $\mu$m, 30 to 50 $\mu$m, 40 to 50 $\mu$m, 500 nm to 40 $\mu$m, 1 to 30 $\mu$m, and 10 to 20 $\mu$m.

The microbubble size is in particular about 5 $\mu$m.

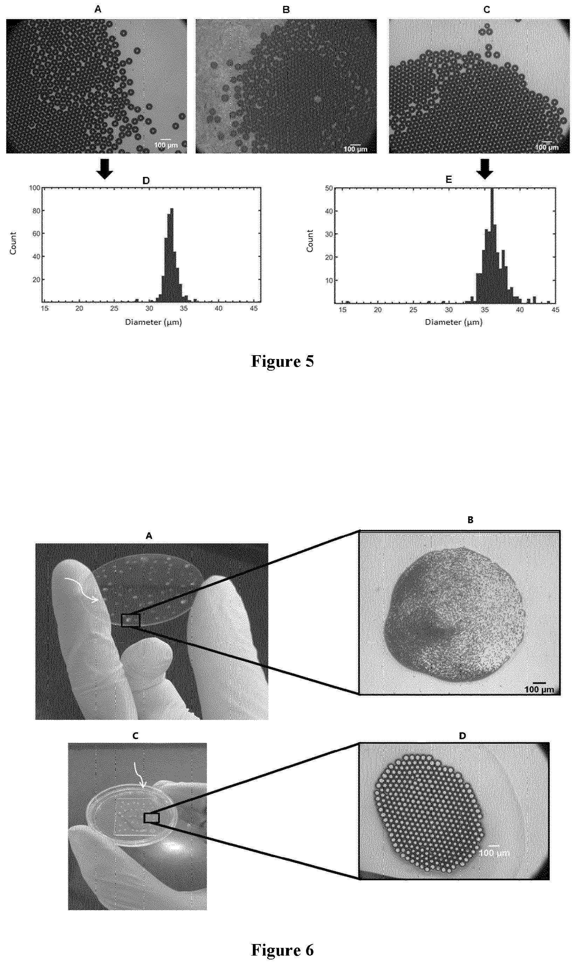

The term *"monolayer",* within the context of the present invention, refers to a single layer of microbubbles on a hydrophilic surface. Each individual bubble is adjacent to at least another bubble present in the monolayer. Depending on the size of the microbubbles, each individual microbubble in the monolayer might, or might not, touch, or be in contact, with an adjacent microbubble. Thus, microbubbles having a size of 20 to 50 $\mu$m comprised in a monolayer are arranged in a "closely packed" configuration. (similar to the one shown in **Figure 6D**), whereas a monolayer comprised of microbubbles having a size of 200 nm to 20 $\mu$m is less densely packed, which means that there can be a space between two adjacent microbubbles (as shown in **Figure 6B**).

**Figure 6D** shows a microscopic image of a monolayer of 40 $\mu$m microbubbles, in which the microbubbles are packed in such a way that the exterior of the shell of each bubble touches the exterior of the shell of at least another microbubble.

In contrast, in the case of 5$\mu$m microbubbles as shown in **Figure 6B,** the monolayer is less densely packed, and the individual microbubbles do not necessarily touch an adjacent microbubble.

According to the present invention, "*at least one monolayer*" refers to one or more than one, monolayer(s) being present on the hydrophilic surface, each individual monolayer being adsorbed onto said surface. Multiple monolayers are thus not stacked on top of each other to form a multilayer, but each monolayer is adjacent, but non-contiguous, to at least one other monolayer.

In this respect, the expression *"non-contiguous"* signifies that the boundaries of monolayers do not touch to the boundaries of other monolayers present on the hydrophilic surface. In other words, there are no contact points between the outer surface of the shells of the microbubbles present in one monolayer, and the outer surface of the shells of the microbubbles present in another monolayer.

The expression "*adjacent*" signifies *"being next to"*.

The different monolayers adsorbed on the hydrophilic surface are thus distributed on the hydrophilic surface and separated from each other by spaces not comprising microbubbles. Typically, and as a non-limiting example, for a hydrophilic surface having dimensions of 25 x 75 mm, comprising around 300 monolayers of approximately 1 mm$^2$, the shortest distance between two adjacent monolayers is at least 500 $\mu$m to 1 mm.

Thus, according to a preferred embodiment, each monolayer of microbubbles that is present on the hydrophilic surface is separated from an adjacent monolayer by a distance of at least 10 times, preferably at least 20 times, the average size of the microbubbles comprised within the monolayers.

In other words, each individual monolayer macroscopically represents a "spot" comprising an increased concentration of microbubbles as compared to regions not comprising a monolayer. This feature is illustrated in **Figure 6,** wherein the photographic images **A** and **C** show the presence of multiple monolayers on a surface, each monolayer representing a "*spot*" on said surface.

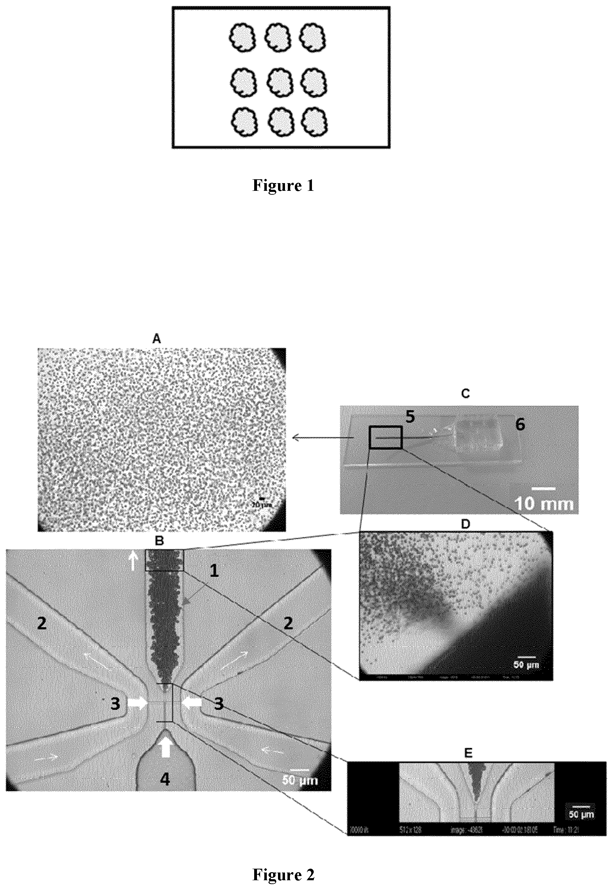

In what follows, the expressions *"monolayer"* and "*spot*" are used, but have the same meaning. **Figure 1** shows a schematic representation of the configuration of nine spots on a surface, clearly showing the spatial separation of the spots.

The term "*adsorbed*" refers to the situation where the microbubbles are attached to the hydrophilic surface through relatively weak interactions, such as electrostatic interactions, or van der Waals forces. For clarity, adsorption does not involve covalent bonding of the microbubbles to the hydrophilic surface.

In a preferred embodiment, the microbubbles are, in addition to being adsorbed to the hydrophilic surface, embedded in a film formed by the shell material.

Thus, **an advantageous embodiment** of the invention is freeze-dried monodisperse microbubbles, wherein:

- the microbubbles comprise a shell and a core,
- the microbubbles have a size inferior to 50 $\mu$m, in particular of from 200 nm to 50 $\mu$m, more in particular of from 1 to 50 $\mu$m, even more in particular of from 1 to 5 $\mu$m,
- the microbubbles have a polydispersity index below 10%, preferably below 6%, more in particular below 5%,

wherein the microbubbles are physiologically inert,

wherein the microbubbles are adsorbed on a hydrophilic surface in the form of at least one monolayer and are embedded in a film formed by the shell material,

said shell material being preferably polyvinyl alcohol.

In this embodiment, the spaces separating the individual microbubbles comprised within each monolayer, are covered with a film formed by the shell material. Thus, in each monolayer, the hydrophilic surface is covered by microbubbles and by said shell material.

The different monolayers present on the hydrophilic surface are separated from each other by regions devoid of microbubbles and devoid of said film of shell materials. In other words, the different monolayers are separated by regions wherein the hydrophilic surface is exposed to the external environment. As is the case with the microbubbles, the film of shell material is also absorbed to the hydrophilic surface, through non-covalent interactions.

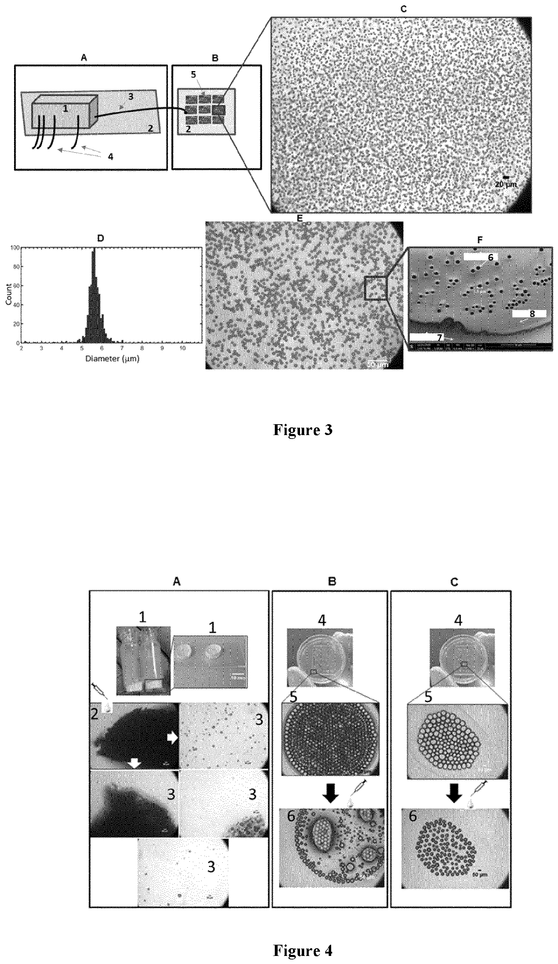

This embodiment is illustrated by **Figure 3F,** in which a polyvinyl alcohol film **8** is present on a hydrophilic surface **7.** The microbubbles **6** are shown to be embedded in the film of shell material, being polyvinyl alcohol.

By *"core"* is meant the volume inside the microbubble, delimited by the inner surface of the shell and comprised of a specific material, different from the shell material. The core being surrounded by the shell. With respect to the microbubbles according to the present invention, the core material comprising the core is a gaz.

By "*shell*" is meant the material completely surrounding the core.

The expression *"physiologically inert"* refers to a material or chemical component that does not produce an adverse physiological reaction when administered to a subject, or a patient. Adverse physiological reactions include, for example, allergic and other systemic reactions, or local inflammation and toxicity.

The freeze-dried monodisperse microbubbles of the invention, as well as the suspension of the invention, are stable.

Thus, in another embodiment of the invention, the freeze-dried monodisperse microbubbles, in the form of at least one monolayer, are stable for at least six 6 months when stored at room temperature in air, in particular for at least 12 months.

Under these conditions less than 10%, in particular less than 1%, of the microbubbles lose their ability to be used as a contrast agent.

In another embodiment of the invention, the shell of the freeze-dried monodisperse microbubbles, in the form of at least one monolayer, is permeable to gaz.

Under vacuum, in particular at pressures below 10 mbar, the gas comprised in the core of the microbubbles can diffuse through the shell and exit the microbubbles. Alternatively, when placed in an external environment comprising a different gas than the one comprised in the core, an exchange of gazes can occur, whereby the gas of the external environment substitutes the gas of the core.

Thus, when microbubbles bubbles comprising PFC in the core, are placed in solutions saturated in air, the air can enter the core through the gas-permeable shells faster than PFC leaves the core, because PFC/SF$_6$ is heavier than air, leading to increased internal pressure in the bubble which can lead to the microbubbles to dilate.

In contrast, if the PFC loaded microbubbles are placed in a gas deficient environment, such as blood, the gas from the core can diffuse out and the microbubble can dissolve in the environment in which it is comprised. This is useful in vivo, because it prevents capillary blockage. Then, escaped, dissolved, gas is eliminated from the body through exhaled breath by the lungs (S. Podell et. al.,Physical and biochemical stability of Optison®, an injectable ultrasound contrast agent, Biotechnol. Appl. Biochem. (1999) 30, 213-223).

In another embodiment, the present invention concerns freeze-dried monodisperse microbubbles as previously described, wherein the shell of the microbubbles has a thickness of from 50 to 100 nm, as measured by scanning electron microscopy.

With the expression *"50 to 100 nm"* is also meant the following ranges: 50 to 90 nm, 50 to 80 nm, 50 to 70 nm, 50 to 60 nm, 60 to 100 nm, 70 to 100 nm, 80 to 100 nm, 90 to 100 nm, 55 to 95 nm, 60 to 80 nm and 65 to 75 nm.

The present invention also relates to freeze-dried monodisperse microbubbles, wherein:

- the microbubbles comprise a shell and a core,
  said shell in particular having a thickness of from 50 to 100 nm, as measured by scanning electron microscopy,
- the microbubbles have a size inferior to 50 $\mu$m, in particular of from 200 nm to 50 $\mu$m, more in particular of from 1 to 50 $\mu$m, even more in particular of from 1 to 5 $\mu$m,
- the microbubbles have a polydispersity index below 10%, preferably below 6%, more in particular below 5%,

wherein the microbubbles are physiologically inert,
wherein the microbubbles are adsorbed on a hydrophilic surface in the form of at least one monolayer,
in particular wherein the microbubbles are embedded in a film formed by the shell material.

In yet another embodiment, the present invention concerns freeze-dried monodisperse microbubbles as previously described, wherein the shell of the microbubbles comprises, or consists of:

- a surfactant, in particular chosen from:

  - an amphiphilic polymer,
  - water-soluble proteins, or
  - phospholipids,

  or mixtures of said polymer, said water-soluble-proteins, and said phospholipids, and/or

- a cryoprotectant.

The expression *"amphiphilic polymer"* is understood to mean a polymer which comprises at least one hydrophilic part and at least one hydrophobic part.

The expression *"water-soluble proteins"* refers to proteins having an aqueous solubility at 25 °C of more than 10 g/L, more in particular more than 40 g/L.

The expression *"cryoprotectant"* refers to a compound used to protect the microbubbles from physical or chemical decay during freezing. Without being bound to theory, the cryoprotectant forms a solid coating that protects the microbubbles from ice crystals that can form during freezing, and from forces associated with exposing the microbubbles to high vacuum during freeze-drying.

In yet another embodiment, the present invention concerns freeze-dried monodisperse microbubbles as previously described, wherein the amphiphilic polymer is both the surfactant and the cryoprotectant.

According to this embodiment, the surfactant present in the shell also has cryoprotecting properties. This is for example, and in particular, the case for the polymer "polyvinyl alcohol", or "PVA". In this situation, an additional surfactant, or an additional cryoprotectant is not necessarily, but can be present in the shell.

In yet another embodiment, the present invention concerns freeze-dried monodisperse microbubbles as previously described, wherein the amphiphilic polymer is a water-soluble amphiphilic polymer chosen from the group consisting of:

polyvinyl alcohol (PVA), polyvinylpyrrolidone (PVP), poly(lactic-co-glycolic acid) (PLGA) and chitosan,
or mixtures of said amphiphilic polymers,
the amphiphilic polymer preferably being polyvinyl alcohol.

In yet another embodiment, the present invention concerns freeze-dried monodisperse microbubbles as previously described, wherein the water-soluble protein is serum albumin.

In yet another embodiment, the present invention concerns freeze-dried monodisperse microbubbles as previously described, wherein the phospholipid is chosen from the group consisting of:

1,2-dipalmitoyl-sn-glycero-3 -phosphate (DPPA), 1,2-dipalmitoyl-sn-glycero-3 - phosphocholine (DPPC), 1,2-dipalmitoyl-sn-glycero-3-phosphocholine (DSPC), 1,2-distearoyl-sn-glycero-3-phosphoethanolamine (DSPE),
or mixtures of said phospholipids,
the phospholipid preferably being DPPA.

In yet another embodiment, the present invention concerns freeze-dried monodisperse microbubbles as previously described, wherein the cryoprotectant is chosen from the group consisting of:

- hydrophilic polymers, in particular hydrophilic polymers comprising hydroxyl groups, more in particular hydrophilic polymers chosen from the group consisting of polyvinyl alcohol (PVA), polyvinylpyrrolidone (PVP), poly(lactic-co-glycolic acid) (PLGA) and chitosan,

- sugars,

the cryoprotectant preferably being polyvinyl alcohol.

Examples of sugars that can be used in the present invention are trehalose, sucrose, glucose, and fructose.

In yet another embodiment, when the microbubbles are embedded in a film formed by the shell material, the shell material is present in the shell of the microbubbles at a ratio of approximately 1:200000 w/w with respect to the total weight of the shell material comprised in the film and in the shell of the microbubbles.

In this embodiment, ratio refers to the cumulative weight of shell materials comprised in the sum of all microbubbles present in a monolayer, compared to all the shell material comprised in the monolayer, both in the film and in the shells. With *"approximately 1:200000"* is meant a value comprised from 100:200000 to 1:100000

In yet another embodiment, the present invention concerns freeze-dried monodisperse microbubbles as previously described, wherein the core of the microbubbles comprises or consists of a gas.

The gas comprised in the core is preferably a hydrophobic gas, in particular a gas chosen from: air, $SF_6$, or perfluorinated hydrocarbons, or mixtures of these gases,

in particular perfluorohexane, or a mixture of perfluorohexane and $SF_6$.

In yet another embodiment, the invention relates to freeze-dried monodisperse microbubbles as previously described, wherein the shell of the microbubbles comprises, or consists of:

- a surfactant, in particular chosen from:

  - an amphiphilic polymer,
  - water-soluble proteins, or
  - phospholipids,
  or mixtures of said polymer, said water-soluble-proteins, and said phospholipids,
  and/or

- a cryoprotectant,

wherein said amphiphilic polymer is in particular both the surfactant and the cryoprotectant,
and/or wherein the core of the microbubbles comprises or consists of a gas, preferably a hydrophobic gas.

In yet another embodiment, the invention relates to freeze-dried monodisperse microbubbles as previously described, wherein the amphiphilic polymer is a water-soluble amphiphilic polymer chosen from the group consisting of:

polyvinyl alcohol (PVA), polyvinylpyrrolidone (PVP), poly(lactic-co-glycolic acid) (PLGA) and chitosan,
or mixtures of said amphiphilic polymers,
the amphiphilic polymer preferably being polyvinyl alcohol,

wherein the water-soluble protein is serum albumin,
wherein the phospholipid is chosen from the group consisting of:

1,2-dipalmitoyl-sn-glycero-3 -phosphate (DPPA), 1,2-dipalmitoyl-sn-glycero-3 - phosphocholine (DPPC), 1,2-dipalmitoyl-sn-glycero-3-phosphocholine (DSPC), 1,2-distearoyl-sn-glycero-3-phosphoethanolamine (DSPE),
or mixtures of said phospholipids,
the phospholipid preferably being DPPA;

wherein the cryoprotectant is chosen from the group consisting of:

- hydrophilic polymers, in particular hydrophilic polymers comprising hydroxyl groups, more in particular hydrophilic polymers chosen from the group consisting of polyvinyl alcohol (PVA), polyvinylpyrrolidone (PVP), poly(lactic-co-glycolic acid) (PLGA) and chitosan,
- sugars,

the cryoprotectant preferably being polyvinyl alcohol,
and/or wherein the gas is chosen from:

air, $SF_6$, or perfluorinated hydrocarbons, or mixtures of these gases,
in particular perfluorohexane, or a mixture of perfluorohexane and $SF_6$.

In yet another embodiment, the present invention concerns freeze-dried monodisperse microbubbles as previously described, wherein the hydrophilic surface is a glass surface, in particular a sodium silicate, or boro silicate glass surface.

The hydrophilic glass surface is preferably pre-treated with $O_2$ plasma to ensure a clean, etched glass surface to which the microbubbles are adsorbed in an optimal manner.

In yet another embodiment, the present invention concerns freeze-dried monodisperse microbubbles as previously described, wherein the hydrophilic surface is flat or curbed, preferably flat, in particular a flat glass slide.

In yet another embodiment, the present invention concerns freeze-dried monodisperse microbubbles as previously described, wherein the microbubbles are adsorbed on a hydrophilic surface in the form of a single monolayer, said monolayer being continuous, uninterrupted by regions that are devoid of microbubbles.

In this embodiment, a single monolayer is present on the hydrophilic surface, without the presence of multiple spots. The microbubbles can be embedded in a film of shell material, absorbed to the hydrophilic surface. Typically, as a non-limiting example, such a single monolayer, adsorbed on a hydrophilic surface having the dimensions of 25 x 75 mm can comprise from 2 to $10^6$ individual microbubbles depending on the size of each microbubble. Preferably the monolayer comprises about $10^6$ individual microbubbles.

In yet another embodiment, the present invention concerns freeze-dried monodisperse microbubbles as previously described, wherein the microbubbles are adsorbed on a hydrophilic surface in the form of more than one monolayers, in particular of from 2 to 1000 monolayers, preferably about 100 monolayers, said more than one monolayers being non-contiguous with respect to each other.

In this embodiment, multiple monolayers of microbubbles are present on the hydrophilic surface, in the form of spots.

With the expression "*of from 2 to 1000 monolayers*" is also meant the following ranges: of from 2 to 800, 2 to 600, 2 to 400, 2 to 200, 2 to 100, 2 to 50, 2 to 10, 10 to 1000, 50 to 1000, 100 to 1000, 200 to 1000, 400 to 1000, 600 to 1000, 800 to 1000, 50 to 900, 100 to 800, 200 to 600, 300 to 500.

In yet another embodiment, the present invention concerns freeze-dried monodisperse microbubbles as previously described, wherein the distance between a first monolayer and a second monolayer, adjacent and non-contiguous to the first monolayer, is substantially identical to the distance between any one of two adjacent non-contiguous monolayers on the hydrophilic surface.

In this embodiment, the distances between each adjacent monolayer adsorbed to the hydrophilic surface are substantially the same. In other words, the plurality of monolayers are homogeneously distributed on the hydrophilic surface,
wherein said distance is the closest distance between the boundaries of the first and the second monolayer.

In yet another embodiment, the present invention concerns freeze-dried monodisperse microbubbles as previously described, wherein the microbubbles are adsorbed on a hydrophilic surface in the form of at least 100 monolayers, and
wherein the hydrophilic surface has a dimension of approximately 25 by 75 mm.

Typically, as an example, in a configuration wherein 100 monolayers are adsorbed to a hydrophilic surface having the dimensions of 25 x 75 mm, wherein each monolayer in particular comprises from 1 to $10^4$ individual microbubbles. Thus, in this example, a total of about $10^6$ microbubbles is present on the surface, the amount needed for clinical use in applications of the microbubbles as contrast agents.

In yet another embodiment, the present invention concerns freeze-dried monodisperse microbubbles as previously described, wherein each of the monolayer(s) comprises of from 2 to $10^6$ microbubbles, in particular approximately $10^4$ microbubbles.

In yet another embodiment, the present invention concerns freeze-dried monodisperse microbubbles as previously described, wherein the microbubbles are adsorbed on a hydrophilic surface in the form of a single monolayer, said monolayer being continuous, uninterrupted by regions that are devoid of microbubbles,
or wherein the microbubbles are adsorbed on a hydrophilic surface in the form of more than one monolayers, in particular of from 2 to 1000 monolayers, preferably about 100 monolayers, said more than one monolayers being non-contiguous with respect to each other,
in particular wherein the distance between a first monolayer and a second monolayer, adjacent and non-contiguous to the first monolayer, is substantially identical to the distance between any one of two adjacent non-contiguous monolayers on the hydrophilic surface.

**A second object** of the present invention relates to freeze-dried monodisperse microbubbles in the form of a powder, wherein:

- the microbubbles comprise a shell and a core,
- the microbubbles have a size inferior to 50 µm, in particular of from 200 nm to 50 µm, more in particular of from 1 to 50 µm, even more in particular of from 1 to 5 µm,
- the microbubbles have a polydispersity index below 10%, preferably below 6%, more in particular below 5%,

wherein the microbubbles are physiologically inert,
said monodisperse microbubbles being substantially devoid of organic solvents, in particular devoid class 1 and/or class 2 solvents, in particular chloroform or dichloromethane.
According to this embodiment of the invention, the freeze-dried microbubbles are not adsorbed to a hydrophilic surface in the form of a monolayer but exist in the form of a bulk powder consisting of microbubbles.
With the expression *"substantially devoid of organic solvents"* is meant that the microbubbles comprise less than 500 ppm of organic solvent, in particular less than 250 ppm, 100 ppm, 50 ppm, 25 ppm, 10 ppm, preferably less than 1 ppm.
By *"organic solvents"* should be understood solvents, other than the gas comprised in the core of the microbubbles. These organic solvents are typically present as residual solvents resulting from the process by which the microbubbles are prepared. For instance, when using an emulsion-based microbubbles preparation method, an aqueous phase and an organic solvent are used, the result of which is that residual organic solvents can end up in the microbubbles.
Class 1 and/or class 2 refers to the ICG Q3C (R6) guidelines of the European Medicines Agency, version of August 9th, 2019, dealing with sets the guidelines for residual solvents present in pharmaceutical ingredients.
In contrast, the microbubbles according to the present invention can be obtained using microfluidic techniques, involving the use of strictly aqueous media.
Thus, a **third object** of the present invention is a process for preparing freeze-dried microbubbles, wherein the microbubbles:

- comprise a shell and a core,
- have a size inferior to 50 µm, in particular of from 200 nm to 50 µm, more in particular of from 1 to 50 µm, even more in particular of from 1 to 5 µm,
- the microbubbles have a polydispersity index below 10%, preferably below 6%, more in particular below 5%,

wherein the microbubbles are physiologically inert,
wherein the microbubbles are adsorbed on a hydrophilic surface in the form of at least one monolayer,
said process comprising a step of:

- freeze-drying monodisperse microbubbles adsorbed on a hydrophilic surface in the form of at least one monolayer, to obtain freeze-dried monodisperse microbubbles adsorbed on a hydrophilic surface in the form of at least one monolayer,
said step of freeze-drying being in particular carried out for a time of approximately 4 hours.

The invention also relates to a process for preparing freeze-dried microbubbles, wherein the microbubbles:

- comprise a shell and a core,
said shell in particular having a thickness of from 50 to 100 nm, as measured by scanning electron microscopy,
- have a size inferior to 50 µm, in particular of from 200 nm to 50 µm, more in particular of from 1 to 50 µm, even more in particular of from 1 to 5 µm,
- the microbubbles have a polydispersity index below 10%, preferably below 6%, more in particular below 5%,

wherein the microbubbles are physiologically inert,
wherein the microbubbles are adsorbed on a hydrophilic surface in the form of at least one monolayer,
in particular wherein the microbubbles are embedded in a film formed by the shell material,
said process comprising a step of:

- freeze-drying monodisperse microbubbles adsorbed on a hydrophilic surface in the form of at least one monolayer, to obtain freeze-dried monodisperse microbubbles adsorbed on a hydrophilic surface in the form of at least one monolayer,
said step of freeze-drying being in particular carried out for a time of approximately 4 hours,

and optionally, before the step of freeze drying, a step of:

- freezing the monodisperse microbubbles adsorbed on a hydrophilic surface in the form of at least one monolayer, to obtain frozen monodisperse microbubbles adsorbed on a hydrophilic surface in the form of at least one monolayer,

said step of freezing being in particular carried out for a time of approximately 2 hours,
said steps of freeze-drying and/or said step of freezing being in particular carried out at a temperature below the glass transition temperature of the cryoprotectant.

Classic freeze-drying techniques, known to the person skilled in the art can be implemented in the process according to the present invention. A shelf freeze-drier is preferably used, wherein the hydrophilic surface can be placed on a tray, or a shelf. The time necessary to freeze-dry is approximately 4 hours but depends on the amount of sample to be freeze-dried.

In another embodiment, the present invention concerns a process for preparing freeze-dried microbubbles as previously described, wherein the process comprises the steps of:

- collecting monodisperse microbubbles on a hydrophilic surface in the form of at least one monolayer, to obtain monodisperse microbubbles adsorbed on a hydrophilic surface in the form of at least one monolayer,
- freeze-drying monodisperse microbubbles adsorbed on a hydrophilic surface in the form of at least one monolayer, to obtain freeze-dried monodisperse microbubbles adsorbed on a hydrophilic surface in the form of at least one monolayer.

The step of collecting the monodisperse microbubbles on a hydrophilic surface can be carried out by spreading the microbubbles onto the hydrophobic surface. In a typical production process, the tip of the tube from which the microbubbles exit the microfluidic chip, is moved over the hydrophilic surface, allowing the deposition of the microbubbles on said surface (**Figure 3**).

In case multiple monolayers are to be produced, the tip of the tube from which the microbubbles exit the microfluidic chip is briefly, for example for 1 to 3 seconds, contacted with the hydrophilic surface. Upon contact, a monolayer is deposited. Repeating said contact, on a different place on the hydrophilic surface leads to the deposition of a subsequent monolayer. In a typical non-limiting example, during each contacting, a drop of 0.1 $\mu$l, comprising $10^4$ microbubbles, is deposited on the surface. This contacting on a different place on the hydrophilic surface can be achieved either by moving the tip over the hydrophobic surface, or by moving the hydrophilic surface under the tip. During these moving operations, the tip is not in contact with the surface. Each drop, in addition to comprising microbubbles, also comprises residual shell material, in particular cryoprotectant.

Upon freeze-drying, the solvent comprised in the drop is removed, leaving a dried monolayer of microbubbles, and a dried film, or coating of residual shell materials comprised in the drop, on the hydrophilic surface. The result of this is that each at least one monolayer is comprised of microbubbles embedded in a film of freeze-dried shell material.

In another embodiment, the present invention concerns a process for preparing freeze-dried microbubbles as previously described, wherein the process comprises the steps of:

- forming monodisperse microbubbles using a microfluidic chip, in particular a flow-focusing chip, said step of forming monodisperse microbubbles comprising a step of microfluidic bubbling of a gas phase, through a liquid phase, comprising an aqueous solution of a surfactant and/or a cryoprotectant, to obtain monodisperse microbubbles, said gas phase forming the core, and said liquid phase forming the shell,
- collecting the monodisperse microbubbles on a hydrophilic surface in the form of at least one monolayer, to obtain monodisperse microbubbles adsorbed on a hydrophilic surface in the form of at least one monolayer,
- freeze-drying the monodisperse microbubbles adsorbed on a hydrophilic surface in the form of at least one monolayer, to obtain freeze-dried monodisperse microbubbles adsorbed on a hydrophilic surface in the form of at least one monolayer.

In another embodiment, the present invention concerns a process for preparing freeze-dried microbubbles as previously described, wherein the process further comprises:

before the step of freeze-drying or before the optional step of freezing, a step of

- collecting monodisperse microbubbles on a hydrophilic surface in the form of at least one monolayer, to obtain monodisperse microbubbles adsorbed on a hydrophilic surface in the form of at least one monolayer,

and optionally, before the step of collecting, a step of

- forming monodisperse microbubbles using a microfluidic chip, in particular a flow-focusing chip,
  said step of forming monodisperse microbubbles comprising a step of microfluidic bubbling of a gas phase,
  through a liquid phase, comprising an aqueous solution of a surfactant and/or a cryoprotectant,
  to obtain monodisperse microbubbles,
  said gas phase forming the core, and said liquid phase forming the shell.

The step of forming monodisperse microbubbles can be performed using standard microfluidic techniques (Introduction to Microfluidics, Patrick Tabeling, Oxford University Press, 2005). A microfluidic flow-focusing geometry such as shown in **Figure 2B** can for example be used.

In a preferred embodiment, the microbubbles are generated at a frequency of up to 10 kHz, corresponding to a throughput of $6 \times 10^5$ microbubbles per minute.

During freeze-drying, which is performed in vacuum, the gas comprised in the core of the microbubbles might diffuse from the core, due to the gas-permeability of the shell. Then, when the vacuum is removed, gases can rediffuse into the core. Placing the freeze-dried microbubbles in an environment comprising a specific gas, such as air, $SF_6$, or perfluorinated hydrocarbons, or mixtures of these gases, allows for the introduction of this/these gas(es) into the core.

Thus, in a particular embodiment, the process of the present invention further comprises a step of introducing a gas into the core of the microbubbles, wherein the freeze-dried microbubbles are placed in an environment comprising the gas to be introduced in the core.

Thus, when the freeze-dried microbubbles are placed in air, their core, upon removal of the vacuum, fill with air of the environment.

In yet another embodiment, the present invention concerns a process for preparing freeze-dried microbubbles as previously described, wherein the process further comprises, between the step of collecting and the step of freeze-drying, a step of:

- freezing the monodisperse microbubbles adsorbed on a hydrophilic surface in the form of at least one monolayer,
  to obtain frozen monodisperse microbubbles adsorbed on a hydrophilic surface in the form of at least one monolayer,
  said step of freezing being in particular carried out for a time of approximately 2 hours.

In this embodiment, the microbubbles are frozen prior to the freeze-drying step and after the step of collecting the microbubbles. The collected microbubbles are sufficiently humid to be able to be frozen, along with the residual water.

In yet another embodiment, the present invention concerns a process for preparing freeze-dried microbubbles as previously described, wherein the step of collecting the monodisperse microbubbles comprises:

- contacting said hydrophilic surface with the tip of a tube through which said microbubbles exit from the microfluidic chip, to obtain a single monolayer of microbubbles.

In yet another embodiment, the present invention concerns a process for preparing freeze-dried microbubbles as previously described, wherein the step of collecting the monodisperse microbubbles comprises:

- contacting said hydrophilic surface with the tip of the tube through which said microbubbles exit from the microfluidic chip, to obtain a first monolayer of microbubbles,
- shifting the tip of the tube or the hydrophilic surface to a position enabling deposition of a second monolayer of microbubbles adjacent and non-contiguous to the first monolayer of microbubbles, during a further step of contacting said hydrophilic surface with said tip,
  to obtain a second monolayer of microbubbles, adjacent to the first monolayer of microbubbles, said first and second monolayer being non-contiguous,
- optionally repeating the steps of shifting and contacting n times, to obtain (n+1) monolayers, n being an integer greater than 1.

In yet another embodiment, the present invention concerns a process for preparing freeze-dried microbubbles as previously described, wherein the step(s) of contacting said hydrophilic surface with said tip is carried out for a period of 1 to 3 seconds.

In yet another embodiment, the present invention concerns a process for preparing freeze-dried microbubbles as previously described, wherein the step of collecting the monodisperse microbubbles comprises:

- contacting said hydrophilic surface with the tip of a tube through which said microbubbles exit from the microfluidic chip, to obtain a single monolayer of microbubbles,
said step of contacting being in particular carried out for a period of 1 to 3 seconds,

or, wherein the step of collecting the monodisperse microbubbles comprises:

- contacting said hydrophilic surface with the tip of the tube through which said microbubbles exit from the microfluidic chip, to obtain a first monolayer of microbubbles,
  said step of contacting being in particular carried out for a period of 1 to 3 seconds,
- shifting the tip of the tube or the hydrophilic surface to a position enabling deposition of a second monolayer of microbubbles adjacent and non-contiguous to the first monolayer of microbubbles, during a further step of contacting said hydrophilic surface with said tip,
  to obtain a second monolayer of microbubbles, adjacent to the first monolayer of microbubbles, said first and second monolayer being non-contiguous,
- optionally repeating the steps of shifting and contacting n times, to obtain (n+1) monolayers, n being an integer greater than 1.

In yet another embodiment, the present invention concerns a process for preparing freeze-dried microbubbles as previously described, wherein the step of freezing is carried out at a temperature below the glass transition temperature of the cryoprotectant,
in particular at a temperature below -20 °C, in particular at a temperature of approximately -25 °C.

At temperatures below the glass transition temperature of the cryoprotectant, the shell material hardens, offering protection from the destructive action of freezing the microbubbles.

In yet another embodiment, the present invention concerns a process for preparing freeze-dried microbubbles as previously described, wherein the step of freeze-drying is carried out at a temperature below the glass transition temperature of the cryoprotectant,
in particular at a shelf temperature below - 40 °C, in particular at a temperature of -50 °C.

At temperatures below the glass transition temperature of the cryoprotectant, the shell material hardens, offering protection from the destructive action of freeze-drying the microbubbles.

In yet another embodiment, the present invention concerns a process for preparing freeze-dried microbubbles as previously described, wherein the relative difference in the arithmetic average size of the monodisperse microbubbles before the freezing and/or freeze-drying steps, compared to after the freezing and/or freeze-drying step is less than 20%, preferably less than 10%.

Freezing microbubbles leads to shrinkage of the microbubbles. Thus, frozen, and freeze-dried microbubbles are smaller than the microbubbles as collected before the freezing and/or freeze-drying step.

In yet another embodiment, the present invention concerns a process for preparing freeze-dried microbubbles as previously described, wherein the relative difference in the polydispersity index of the microbubbles before the freezing and/or freeze-drying steps, compared to after the freezing and/or freeze-drying step is less than 20%, preferably less than 10%.

With the expression *"less than 20%",* should also be understood: less than 18%, 16%, 14%, 12%, 10%, 8%, 6%, 4%, 2%, in particular less than 1%.

In yet another embodiment, the present invention concerns a process for preparing freeze-dried microbubbles as previously described, wherein the shell of the microbubbles has a thickness of from 50 to 100 nm, as measured by scanning electron microscopy.

In yet another embodiment, the present invention concerns a process for preparing freeze-dried microbubbles as previously described, wherein the liquid phase comprises:

- a surfactant, in particular chosen from:

    ▪ an amphiphilic polymer, or
    ▪ phospholipids,
    or mixtures of said polymer and said phospholipids, and/or

- a cryoprotectant.

The liquid phase further comprises an aqueous component, in which the surfactant and the cryoprotectant are solubilized.

In yet another embodiment, the present invention concerns a process for preparing freeze-dried microbubbles as previously described, wherein the amphiphilic polymer is a water-soluble polymer, chosen from the group consisting of:

polyvinyl alcohol (PVA), polyvinylpyrrolidone (PVP), poly(lactic-co-glycolic acid) (PLGA) and chitosan,
or mixtures of said amphiphilic polymers,
the amphiphilic polymer preferably being polyvinyl alcohol.

In yet another embodiment, the present invention concerns a process for preparing freeze-dried microbubbles as previously described, wherein the phospholipid is chosen from the group consisting of:

1,2-dipalmitoyl-sn-glycero-3 -phosphate (DPPA), 1,2-dipalmitoyl-sn-glycero-3 - phosphocholine (DPPC), 1,2-dipalmitoyl-sn-glycero-3-phosphocholine (DSPC), 1,2-distearoyl-sn-glycero-3-phosphoethanolamine (DSPE),
or mixtures of said phospholipids,
the phospholipid preferably being DPPA.

In yet another embodiment, the present invention concerns a process for preparing freeze-dried microbubbles as previously described, wherein the cryoprotectant is chosen from the group consisting of:

- hydrophilic polymers, in particular hydrophilic polymers comprising hydroxyl groups, more in particular hydrophilic polymers chosen from the group consisting of polyvinyl alcohol (PVA), polyvinylpyrrolidone (PVP), poly(lactic-co-glycolic acid) (PLGA) and chitosan,

- sugars,

the cryoprotectant preferably being polyvinyl alcohol.
In yet another embodiment, the present invention concerns a process for preparing freeze-dried microbubbles as previously described, wherein the amphiphilic polymer is both a surfactant and/or a cryoprotectant, preferably polyvinyl alcohol.
In yet another embodiment, the present invention concerns a process for preparing freeze-dried microbubbles as previously described, wherein the gas phase comprises, or consists of a hydrophobic gas, in particular chosen from:

air, $SF_6$, or perfluorinated hydrocarbons, or mixtures of these gases,
in particular perfluorohexane, or a mixture of perfluorohexane and $SF_6$.

Hydrophobic gas in the sense of the invention are gaseous substances that are not freely miscible with water in the liquid phase
In yet another embodiment, the present invention concerns a process for preparing freeze-dried microbubbles as previously described, wherein the liquid phase comprises:

- a surfactant, in particular chosen from:

  - an amphiphilic polymer,
  - water-soluble proteins, or
  - phospholipids,
  or mixtures of said polymer, said water-soluble-proteins, and said phospholipids,
  and/or

- a cryoprotectant,

wherein said amphiphilic polymer is in particular both the surfactant and the cryoprotectant, preferably polyvinyl alcohol.
and/or wherein the gas phase comprises or consists of a gas, preferably a hydrophobic gas.
In yet another embodiment, the present invention concerns a process for preparing freeze-dried microbubbles as previously described, wherein the amphiphilic polymer is a water-soluble amphiphilic polymer chosen from the group consisting of:

polyvinyl alcohol (PVA), polyvinylpyrrolidone (PVP), poly(lactic-co-glycolic acid) (PLGA) and chitosan,
or mixtures of said amphiphilic polymers,
the amphiphilic polymer preferably being polyvinyl alcohol,
wherein the water-soluble protein is serum albumin,
wherein the phospholipid is chosen from the group consisting of:

1,2-dipalmitoyl-sn-glycero-3 -phosphate (DPPA), 1,2-dipalmitoyl-sn-glycero-3 - phosphocholine (DPPC), 1,2-dipalmitoyl-sn-glycero-3-phosphocholine (DSPC), 1,2-distearoyl-sn-glycero-3-phosphoethanolamine (DSPE), or mixtures of said phospholipids, the phospholipid preferably being DPPA;

wherein the cryoprotectant is chosen from the group consisting of:

- hydrophilic polymers, in particular hydrophilic polymers comprising hydroxyl groups, more in particular hydrophilic polymers chosen from the group consisting of polyvinyl alcohol (PVA), polyvinylpyrrolidone (PVP), poly(lactic-co-glycolic acid) (PLGA) and chitosan,
- sugars, the cryoprotectant preferably being polyvinyl alcohol,

and/or wherein the gas is chosen from:

air, $SF_6$, or perfluorinated hydrocarbons, or mixtures of these gases, in particular perfluorohexane, or a mixture of perfluorohexane and $SF_6$.

In yet another embodiment, the present invention concerns a process for preparing freeze-dried microbubbles as previously described, wherein the hydrophilic surface is a glass surface, in particular a sodium silicate, or boro silicate glass surface.

In yet another embodiment, the present invention concerns a process for preparing freeze-dried microbubbles as previously described, wherein the hydrophilic surface is flat or curbed, preferably flat, in particular a glass slide.

The present invention also concerns a process for producing freeze-dried monodisperse microbubbles, in the form of a powder, wherein the process comprises a step of:

- removing the freeze-dried monodisperse microbubbles in the form of at least one monolayer, as previously defined, from said hydrophilic surface, to obtain monodisperse microbubbles, in the form of a powder.

The microbubbles of the present invention are adsorbed on the hydrophilic surface through relatively weak, non-covalent interactions. Thus, they are susceptible to being removed, for example by carefully scratching them from the surface. A bulk powder of freeze-dried monodisperse microbubbles, can be obtained.

**A fourth object** of the present invention concerns freeze-dried monodisperse microbubbles adsorbed on a hydrophilic surface in the form of a single monolayer, such as obtained by the process for preparing freeze-dried microbubbles as previously described.

**A fifth object** of the present invention concerns freeze-dried monodisperse microbubbles adsorbed on a hydrophilic surface in the form of at least one monolayer, such as obtained by the process for preparing freeze-dried microbubbles as previously described.

**A sixth object** of the present invention concerns freeze-dried monodisperse microbubbles, in the form of a powder, such as obtained by the process as previously described.

**A seventh object** of the present invention is a process for preparing an aqueous suspension of monodisperse microbubbles, wherein the process comprises:

- a step of resuspending freeze-dried monodisperse microbubbles adsorbed on a hydrophilic surface as previously described, to obtain an aqueous suspension of monodisperse microbubbles, wherein the microbubbles are physiologically inert, said step of resuspending comprising: contacting a freeze-dried monolayer of monodisperse microbubbles as previously described with an aqueous medium, preferably a buffer, more preferably phosphate buffered saline.

According to this object of the present invention, the freeze-dried microbubbles are subjected to an aqueous medium, also referred to as resuspension medium, whereby the microbubbles are detached from the surface. An aqueous, milky white suspension of microbubbles is initially the result.

The inventors have observed that said suspension of microbubbles gradually, over time, converts into a solution. Indeed, the permeability of the shell leads to diffusion of the gas which is comprised in the core, through the shell, which can lead to the microbubbles to disintegrate and the materials to solubilize, resulting in the obtention of a solution. This dissolution is not instantaneous, and the aqueous suspension of monodisperse microbubbles according to the present invention is sufficiently stable in air for up to 20 minutes, in particular for up to 10 minutes, more in particular for up to 3 minutes, to be used, for example as a monodisperse contrast agent.

In this respect, the stability also relates to the stability of the monodispersity and the microbubble size, said monodispersity being conserved for up to 20 minutes, After 20 minutes; the microbubbles are susceptible to becoming polydisperse.

Thus, in another embodiment of the invention, the suspension of freeze-dried monodisperse microbubbles, is stable in air for up to 20 minutes, in particular for up to 10 minutes, more in particular for up to 3 minutes, to be used, for example as a monodisperse contrast agent. Under these conditions less than 10%, in particular less than 1%, of the microbubbles lose their ability to be used as a contrast agent, whereby the change in resonance frequency as compared to the initial resonance frequency measured immediately after the preparation of the suspension is less than 1%, in particular less than 0.1%.

In another embodiment, the present invention concerns a process for preparing an aqueous suspension of microbubbles, as previously defined, wherein said step of resuspending is carried out in a microfluidic chip, in particular in a microfluidic chip comprising pillar based microchannels, or comprising slit microchannels.

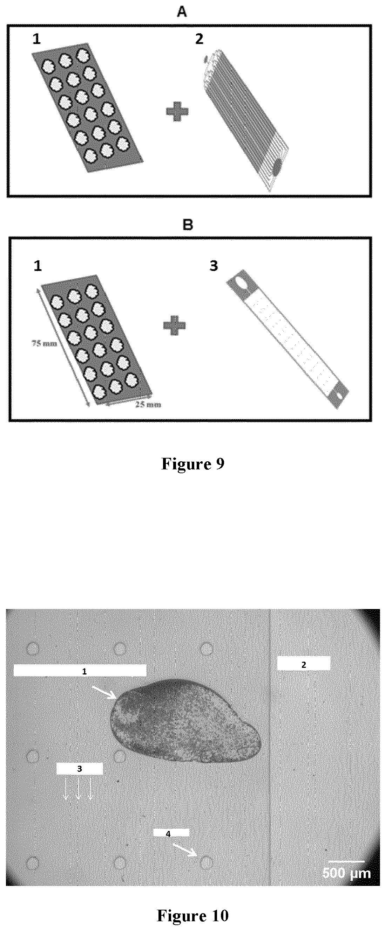

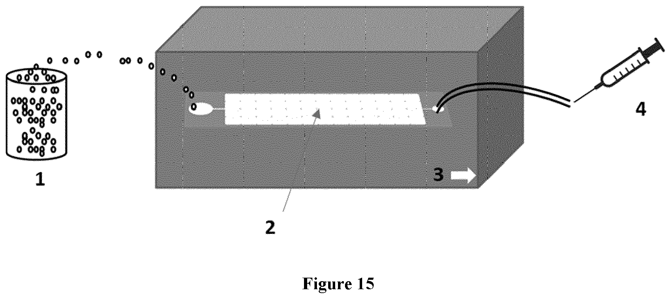

This embodiment is, for example, represented by **Figure 9,** which shows a schematic representation of a microfluidic resuspension chip comprising pillar based or slit based microchannels. The step of resuspending can be performed, in this case, by providing a resuspension medium through the inlet of the microfluidic chip and collecting the suspension through the outlet of the chip, as illustrated in Figure 15.

**An eight object** of the present invention is a process for preparing an aqueous suspension of monodisperse microbubbles, said process comprising a step of resuspending the freeze-dried monodisperse microbubbles, in the form of a powder, as previously defined, to obtain an aqueous suspension of monodisperse microbubbles, wherein the microbubbles are physiologically inert, said step of resuspending comprising:

- contacting of freeze-dried monodisperse microbubbles, in the form of a powder, with an aqueous medium preferably a buffer, more preferably phosphate buffered saline.

In this embodiment, the resuspension is not carried out inside a microfluidic chip, but rather is carried out in a vial comprising a bulk powder of microbubbles.

In a preferred embodiment, the present invention concerns a process for preparing an aqueous suspension of monodisperse microbubbles as previously described, wherein the aqueous medium is saturated with a gas, in particular a gas chosen from $SF_6$, air, or perfluorinated hydrocarbons, in particular perfluorohexane, or a mixture of perfluorohexane and $SF_6$, said gas, or mixture of gases, being preferably the same as the gas, or mixture of gases, comprised in, or constituting the core of said microbubbles.

Saturating the aqueous resuspension medium with a gas prevents loss of the gas that is comprised within the core of the microbubbles by diffusion.

In an even more preferred embodiment, the present invention concerns a process for preparing an aqueous suspension of monodisperse microbubbles as previously described, wherein the aqueous medium is phosphate buffered saline, saturated with a gas chosen from air, perfluorinated hydrocarbons or $SF_6$, wherein the gas pressure is below 1000 kPa.

At gas elevated gas pressures, i.e. over 1000 kPa, Ostwald ripening of the microbubbles can occur.

The present invention also relates to a process for preparing an aqueous suspension of monodisperse microbubbles, wherein the process comprises:

- a step of resuspending freeze-dried monodisperse microbubbles adsorbed on a hydrophilic surface as previously described, to obtain an aqueous suspension of monodisperse microbubbles, wherein the microbubbles are physiologically inert, said step of resuspending comprising: contacting a freeze-dried monolayer of monodisperse microbubbles as previously described with an aqueous medium, preferably a buffer, more preferably phosphate buffered saline,

said step of resuspending being in preferably carried out in a microfluidic chip, in particular in a microfluidic chip comprising pillar based microchannels, or comprising slit microchannels. said aqueous medium being in particular saturated with a gas, in particular a gas chosen from $SF_6$, air, or perfluorinated hydrocarbons, in particular perfluorohexane, or a mixture of perfluorohexane and $SF_6$, said gas, or mixture of gases, being preferably the same as the gas, or mixture of gases, comprised in, or constituting the core of said microbubbles.

**A ninth object** of the present invention relates to an aqueous suspension of monodisperse microbubbles, such as obtained by the process as previously described.

**A tenth object** of the present invention relates to an aqueous suspension of monodisperse microbubbles, wherein:

- the microbubbles comprise a shell and a core,
- the microbubbles have a size inferior to 50 $\mu$m, in particular of from 200 nm to 50 $\mu$m, more in particular of from 1 to 50 $\mu$m, even more in particular of from 1 to 5 $\mu$m,
- the microbubbles have a polydispersity index below 10%, preferably below 6%, more in particular below 5%,

wherein the microbubbles are physiologically inert,
wherein the monodisperse microbubbles are substantially devoid of organic solvents, in particular devoid class 1 and/or class 2 solvents, in particular chloroform or dichloromethane,
wherein the aqueous suspension comprises an aqueous medium in the form of a buffer, preferably phosphate buffered saline,
wherein the shell preferably comprises polyvinyl alcohol, and wherein the core preferably comprises a hydrophobic gas.

In another embodiment, the present invention concerns an aqueous suspension of monodisperse microbubbles as previously defined, wherein the concentration of microbubbles is comprised from 0.1to 20 w/w% compared to the total weight of the aqueous suspension.

With the expression "0.1 to 20 w/w%" relating to the concentration of microbubbles in the aqueous suspension, should also be understood the following ranges: 0.1 to 15 w/w%, 0.1 to 10 w/w%, 0.1 to 5 w/w%, 1 to 20 w/w%, 5 to 20 w/w%, 10 to 20 w/w%, 15 to 20 w/w%, 1 to 15 w/w%, 5 to 10 w/w%, in particular 8 w/w%.

The freeze-dried monodisperse microbubbles according to the present invention present interesting acoustic properties, due to their high level of monodispersity. These unprecedented acoustic properties can be exploited in several fields.

The present invention also concerns an aqueous suspension of freeze-dried monodisperse microbubbles, such as obtained by the process for preparing an aqueous suspension of monodisperse microbubbles, as previously described, or an aqueous suspension of monodisperse microbubbles, wherein:

- the microbubbles comprise a shell and a core,
  said shell in particular having a thickness of from 50 to 100 nm, as measured by scanning electron microscopy,
- the microbubbles have a size inferior to 50 $\mu$m, in particular of from 200 nm to 50 $\mu$m, more in particular of from 1 to 50 $\mu$m, even more in particular of from 1 to 5 $\mu$m,
- the microbubbles have a polydispersity index below 10%, preferably below 6%, more in particular below 5%,

wherein the microbubbles are physiologically inert,
wherein the monodisperse microbubbles are substantially devoid of organic solvents, in particular devoid class 1 and/or class 2 solvents, in particular chloroform or dichloromethane,
wherein the aqueous suspension comprises an aqueous medium in the form of a buffer, preferably phosphate buffered saline,
wherein the shell preferably comprises polyvinyl alcohol, and wherein the core preferably comprises a hydrophobic gas.

Thus, **an eleventh object** of the present invention relates to the use of the suspension of freeze-dried monodisperse microbubbles as previously described, or of the freeze-dried monodisperse microbubbles as previously described:

- as an ultrasound contrast agent for molecular imaging,
- as an ambient pressure sensor in non-invasive blood-pressure measurements,
- as ultrasound agents for sonoporation,
- as acoustic biosensors,

in particular as an ultrasound contrast agent.

The use of microbubbles for acoustic purposes, such as contrast agent or as ultrasound agents, is subject to several sources of noise that degrades the quality of the image, such as diffusion and speckle.

Clinical ultrasound scanners operate at a narrow frequency bandwidth (typically between 2.5 and 7.5 MHz), and only a small fraction of the microbubbles participates in the generation of the echo. The techniques that would make it possible to improve the signal to noise ratio, or the efficiency quantified by STAR (diffusion to attenuation ratio), by exploiting the harmonic or subharmonic components of the echo, are difficult to implement to commercial microbubbles due to their polydispersity.

Thus, the monodisperse microbubbles according to the present invention present particular advantages in terms of the ability to improve the signal to noise ratio, and their ability to show a well-defined sharp resonance frequency, and are thus of particular interest for use in for acoustic purposes.

In another embodiment, the present invention concerns the use of monodisperse microbubbles as previously described, wherein the microbubbles give an acoustic response at a mechanical index MI lower than 0.4, in response to a driving acoustic pulse.

In another embodiment, the present invention concerns the use of monodisperse microbubbles previously described, wherein the resonance frequency response is comprised from 0,14 MHz to 251,8 MHz, in particular being approximately 1.945 MHz for 5$\mu$m microbubbles having a polydispersity index of 5%.

The resonance frequency response of the microbubbles being inversely proportional to the microbubble size, according to Minnaert's law,

wherein Minnaert's law is defined by Formula 2:

$$w_o = \sqrt{\frac{3\kappa P_o}{\rho R_o^2}}$$

**Formula 2**

wherein:

$\omega_0$ = Bubble oscillation eigenfrequency
$\kappa$ = Polytropic exponent of microbubble filling gas
$P_0$ = The ambient pressure
$\rho$ = The liquid density
R = Radius of the bubble at rest

In case of heavy gases, having a density higher than the density of air; such as perfluorohydrocarbons, $\kappa$ = 1.1, $\rho$ = 1000 kg/m$^3$ and $P_0$ = 100 kPa, the above Formula 2 can be rewritten into **Formula 3** below:

$$f_o R_o = 3.3 \; \mu m \, MHz$$

**Formula 3**

wherein:

$F_o$ = resonance frequency of the non-coated bubbles

**An eleventh object** of the present invention is a microfluidic chip comprising:

- inside the chip, a hydrophobic surface, preferably a glass slide, on which monodisperse microbubbles are absorbed in the form of at least one monolayer, as previously described,
- one or more inlets for supplying the aqueous medium
- one or more outlets for collecting the resuspended microbubbles.

A typical, non-limiting example of such an arrangement is shown in **Figure 15,** in which **5** represents a microfluidic chip comprised in a pillar-based PDMS microfluidic system. The microfluidic chip can be stored and the microbubbles can be resuspended when needed, by injecting the resuspension medium through the inlet of the chip, and collecting the suspension of microbubbles on the outlet by transferring it into for example a sterile vial.

The following figures and examples illustrate the implementation of the present invention in some of its embodiments, the figures and examples should not be construed as limiting the scope of the invention.

**FIGURES**

**Figure 1** schematically represents the concept of multiple monolayers. The figure shows nine monolayers that are arranged on a hydrophilic surface in the form of "spots".

**Figure 2** represents the microfluidic generation of 5 $\mu$m (CV < 5%) PVA microbubbles according to examples 1 and 3.

**Fig 2A** shows an optical microscopy image of the microbubbles as formed. **Fig 2B** shows an outtake of the part of the microfluidic chip where the microbubbles are formed, wherein **1** represents the microbubbles, **2** represents the anti-clogging system, **3** represents the continuous phase, or liquid phase, and **4** represents the entry of the gas phase. **Fig 2C** is a representation of the microfluidic chip used for bubble formation, wherein **5** represents the lateral collection tubing, and **6** represents the inlets and the outlets. **Fig 2D** shows an outtake of the part of the chip, where the outlet/collection is located. **Fig 2E** shows an outtake of the part of the microfluidic chip where the microbubbles are formed.

**Figure 3** illustrates the experimental setup for the production of the monolayer, according to example 4.

**Fig 3A** represents the experimental setup for microfluidic bubble production, wherein **1** represents the PDMS chip, **2** represents the hydrophilic surface, being a glass slide, **3** represents the collection tubing, and **4** represents the inlet and outlet tubing. **Fig 3B** represents the collection of microbubbles in the form of a monolayer on a plasma treated glass slide **2,** wherein **5** represents the monolayers of microbubbles, in the form of spots, and **6** represents the hydrophilic surface. **Fig 3D** shows the size distribution of the microbubbles obtained, as determined by SEM. An average size of 5.64 $\pm$ 0,37 $\mu$m is observed, with a CV of 6,5%. The x-axis represents the diameter of the bubble expressed in $\mu$m, and the y-axis represents the bubble count. **Fig's 3C and 3E** correspond to optical images of the freeze-dried monolayer of microbubbles. **Fig 3E** shows a SEM image of the freeze-dried microbubbles, wherein **6** represents ruptured microbubbles, **7** represents the silicon substrate and **8** represents the dried PVA film.

**Figure 4** represents the freeze-drying and hydration of bulk, multilayer, and monolayer microbubbles. **Fig 4A** shows images of freeze-dried bulk microbubbles, wherein **1** represents a photographic image of a sample of freeze-dried microbubble powder, referred to as the freeze-cake, **2** represents an optical microscope image of the freeze-dried bulk microbubbles, whereas **3** represent four optical microscope images of resuspended bulk microbubbles. **Fig 4B** shows images of freeze-dried multilayers of microbubbles. **4** represents a photographic image of multilayers of freeze-dried microbubbles adsorbed on a glass slide. **5** represents an optical microscopy image of a freeze-dried multilayer of microbubbles and **6** represents an optical microscopy image of resuspended freeze-dried microbubbles. **Fig 4C** shows images of freeze-dried monolayers of microbubbles. **4** represent a photographic image of freeze-dried monolayers of microbubbles adsorbed on a glass slide. **5** represents an optical microscopy image of a freeze-dried monolayer of microbubbles and **6** represents an optical microscopy image of resuspended freeze-dried microbubbles.

**Figure 5** represents optical images of a monolayer of microbubbles before freezing, frozen and freeze-thawed. **Fig 5A** shows an optical image of microbubbles before freeze-drying, having an average size of 33 $\pm$ 2 $\mu$m, with a CV of 6%, as shown in **Fig 5D** that represents the size distribution of these microbubbles. **Fig 5B** shows an optical image of frozen microbubbles at room temperature. **Fig 5C** shows an optical image of freeze-thawed microbubbles, having an average size of 36 $\pm$ 3 $\mu$m, with a CV of 8%, as shown in **Fig 5E** that represents the size distribution of these microbubbles. In **Fig 5D** and **Fig 5E,** the x-axes represent the diameter of the bubble expressed in $\mu$m, and the y-axes represent the bubble count.

**Figure 6** represents images that show freeze-dried microbubbles in the form of a monolayer according to example 4. **Fig 6A** is a macroscopic image of a glass slide comprising monolayers of freeze-dried 5 $\mu$m microbubbles. **Fig 6B** shows an outtake of **Fig 6B. Fig 6C** is a macroscopic image of a glass slide comprising monolayers of freeze-dried 5 $\mu$m microbubbles. **Fig 6D** shows an outtake of **Fig 6C.**

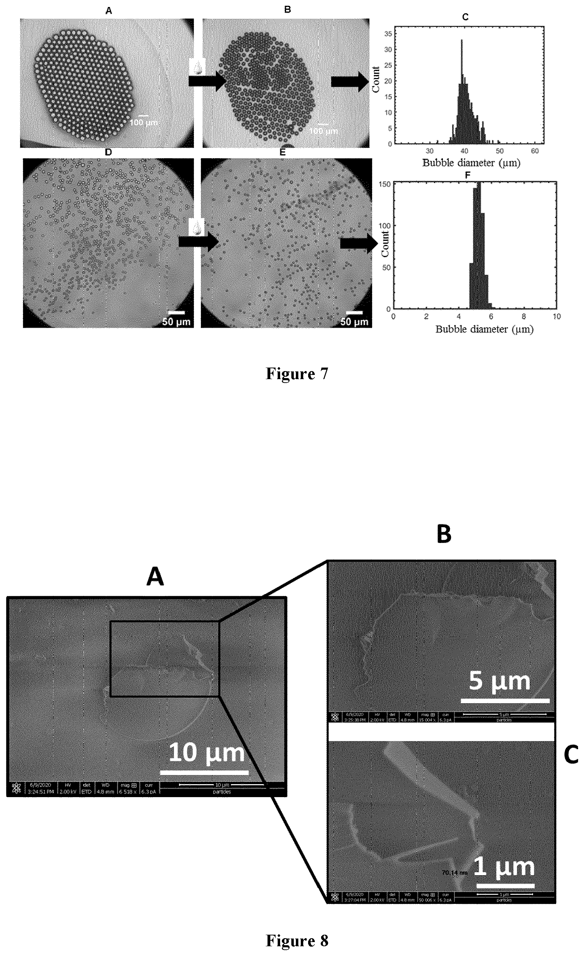

**Figure 7** represents the resuspension of the freeze-dried microbubbles according to example 5. **Fig 7A** shows an optical image of a monolayer of freeze-dried 40 $\mu$m microbubbles. **Fig 7B** shows the optical image of the resuspended microbubbles, having an average size of 40,9 $\pm$ 3,5 $\mu$m, with a CV of 8,6%, as shown in **Fig 7C. Fig 7D** shows an optical image of a monolayer of freeze-dried 5 $\mu$m microbubbles. **Fig 7E** shows the optical image of the resuspended microbubbles, having an average size of 5,2 $\pm$ 0,2 $\mu$m, with a CV of 3,9%, as shown in **Fig 7C.** In **Fig 7C** and **Fig 7F,** the x-axes represent the diameter of the bubble expressed in $\mu$m, and the y-axes represent the bubble count.

**Figure 8** represents SEM images **Fig's 8A, 8B** and **8C** of freeze-dried PVA microbubbles, used to measure the shell thickness according to example 5.

**Figure 9** represents a schematic representation of microfluidic-based resuspension systems, as used in example 6, wherein **Fig 9A** shows a slit based resuspension system **2,** and **Fig 9B** shows a pillar based resuspension system **3.** In both Figures, **1** represents a freeze-dried monolayer of microbubbles.

**Figure 10** represents an optical image of a monolayer of freeze-dried microbubbles, according to example 6, in the form of a spot, sealed in a pillar-based microfluidic resuspension system. Wherein **1** represents a freeze-dried monolayer of microbubbles, **2** represents the glass slide, **3** represents the liquid flow and **4** shows a pillar.

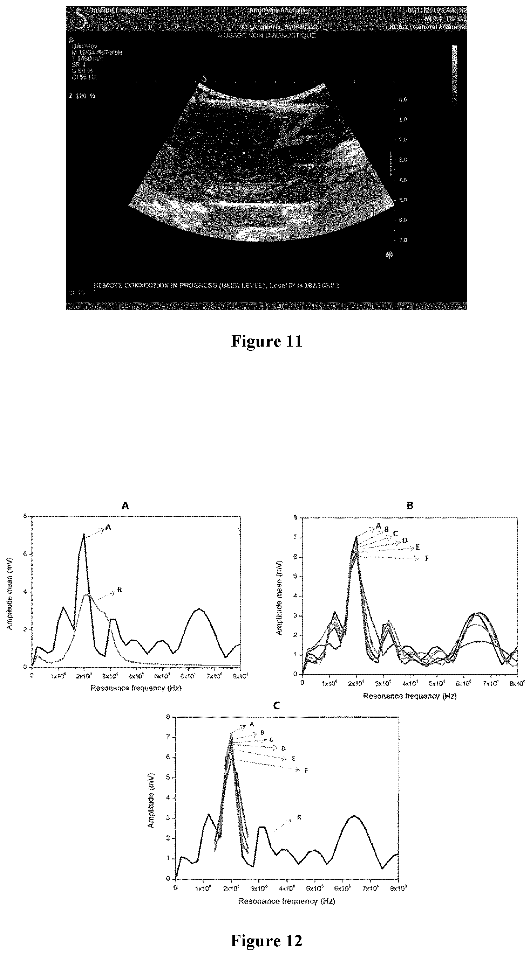

**Figure 11** represents an ultrasound image of microbubbles *in vitro,* obtained according to example 7. The arrow points to acoustically sensitive microbubbles floating in a PBS solution.

**Figure 12** represents the mean of 1000 measurements according to example 8.

**Fig12A** shows the resonance frequency response of microbubbles (A) versus reference measurement (R). **Fig 12B** shows the comparison for the mean of six different measurements, A, B, C, D, E and F. **Fig 12C** shows Gaussian-fitted curves of measurements, A, B, C, D, E and F, and of R, corresponding to measurement A in Fig 12A. In Fig 12A, 12B and 12C, the x-axis corresponds to the resonance frequency (Hz) and the y-axis corresponds to the mean amplitude (mV).

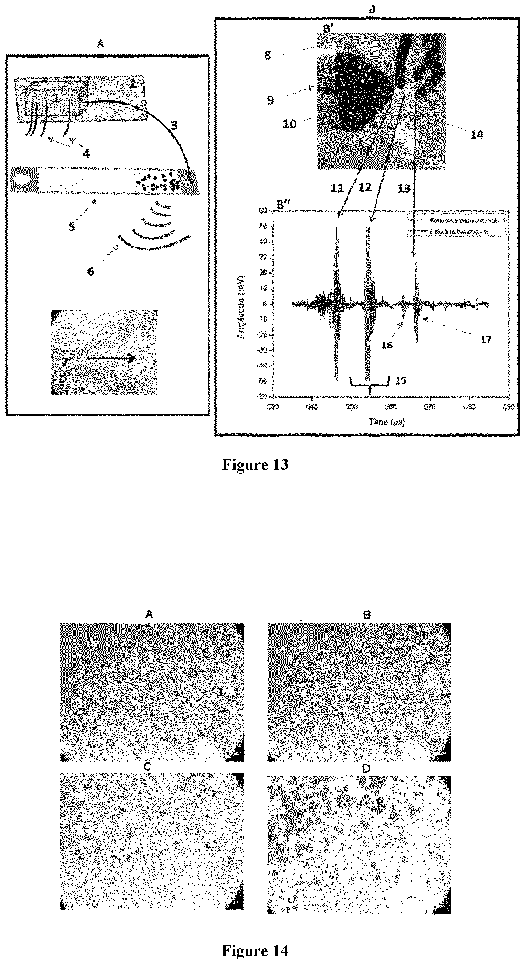

**Figure 13** represents the acoustic characterization of stationary bubbles, comprised in the chip. **Fig 13A** represents the experimental setup for the complete process of microbubble production, resuspension and use as a contrast agent. **1** represents the PDMS chip, **2** represents the hydrophilic surface, being a glass slide, **3** represents the collection tubing, and **4** represents the inlet and outlet tubing. **5** represents a pillar-based resuspension system having a height of 50 $\mu$m, **6** represents the acoustic signal. **7** represents an outtake of chip inlet of the resuspension system.

**Fig 13B** represents the experimental set-up (Fig13B') and an echo response of the system (Fig13B"), wherein **8** represents the acoustic gel, **9** represents the transducer, **10** represents the 3D printed waveguide, **11** represents the echo front wall, **12** represents the echo microchannels, **13** the echo front wall, **14** represents the PDMS-PDMS bonded bubble reservoir, **15** represents the area used for the resonance frequency measurements, **16** represents the curve obtained for the reference measurement and **17** represents the curve obtained for the resonance frequency measurement of the microbubbles in the chip. In Fig13B", the x-axis represents the time ($\mu$s) and the y-axis represents the amplitude (mV).

**Figure 14** represents optical microscopy images show the time-dependent size distribution of stationary bubbles in the chip in consecutive acoustic experiments according to example 8, and Figure 12. **Fig 14A** shows the microbubbles after 1 minute, wherein **1** represents a pillar. **Fig14B** shows the microbubbles after 3 minutes, **Fig 14C** shows the microbubbles after 10 minutes, **Fig14D** shows the microbubbles after 20 minutes.

**Figure 15** represents a chip-based resuspension system, wherein **1** represents Resuspended ready-to-inject monodisperse bubbles in a sealed glass vial, **2** represents a pillar-based PDMS microfluidic system, **3** represents a gas-filled enclosed packaging and **5** represents a liquid injection of resuspension medium.

**EXEMPLES**

**Abbreviations and definitions**

| | |
|---|---|
| CMC | Critical Micelle Concentration |
| CV | Coefficient of Variation |
| PBS | Phosphate-Buffered Saline |
| PDI | Poly Dispersity Index |
| PDMS | Polydimethylsiloxane |
| PFC | Perfluorocarbon |
| PVA | Poly Vinyl Alcohol |
| SEM | Scanning Electron Microscopy |
| Tg | Glass transition Temperature |

**General remarks**

Below, monodisperse PVA-shelled microfluidic microbubbles (PDI<5%) were prepared, encapsulating perfluorocarbon (PFC), or Sulfur hexafluoride ($SF_6$) gas, with polydimethylsiloxane (PDMS)-based microfluidic chips. The problem of long-term storage and transportation, without monodispersity degradation, has been solved by freeze-drying the microbubbles in the form of a monolayer and in the presence of a cryoprotectant.

The polydispersity index is calculated according to **Formula 1.**

$$PDI = \frac{100\ \sqrt{\langle d^2 \rangle - \langle d \rangle^2}}{\langle d \rangle} \qquad \textbf{Formula 1}$$

in which

d represents the diameter of the individual microbubbles as measured by optical microscopy.
<d> represents the average of the measured diameter for a population of microbubbles.

**EXAMPLE 1: Microfluidic chip preparation**

The molds for the PDMS chips were fabricated using conventional soft lithography and prepared using replica-molding techniques (J. Cooper McDonald et al., Fabrication of microfluidic systems in poly(dimethylsiloxane), Electrophoresis 2000, 21, p.27-40). Then, the molds were cut, inlets and outlets were punched through the PDMS, and microfluidic channels sealed with a microscopic glass slide by the application of $O_2$ plasma with a plasma cleaner (The CUTE, Femto Science Inc., Republic of Korea), which tunes hydrophilicity of the channels as well. Then, plastic tubings were connected to inlet and outlets of the device.

The microfluidic chip obtained contains two liquid inlets and two corresponding fluid outlets, from which excessive material can be recovered, one gas inlet and one outlet, from which the microbubbles are collected. The outlet includes a collecting tube, aligned along the axis of the collecting channel to avoid coalescence phenomena (e.g., lateral collection), and an entry (e.g., pusher), allowing to increase the flow-rate of the continuous phase, i.e. the liquid phase comprising the shell materials, and thereby tune the bubble concentration, along with their interactions. This step favors the high throughput and reliability of the process. The pusher is helpful, but it is not guaranteed that it is critical. The microfluidic channels consist of the combination of flow-focusing geometry, whose width and height are variable (from 5 μm, as in **Figure 1,** to 100 μm), and step-emulsification, which enlarges the dimension with an upward step-like channel whose height is around 20 μm, in order to reduce the hydrodynamic resistance. An anti-clogging system (so-called river channels) was included to avoid large aggregates or contaminants to clog the channels to ensure a prolonged stable generation (Malloggi et al., Monodisperse Colloids Synthesized with Nanofluidic Technology, Langmuir. 26 (2010) 2369-2373).

**EXAMPLE 2: Microbubble formulation**

The gas-phase of the system, which defines the gas core of the microbubbles, was perfluorohexane ($C_6F_{14}$), or $SF_6$ to enhance the lifetime of the microbubbles as the permeable shell leads to a rapid escape of soluble gases such as air.

Thus, gases that are highly hydrophobic, less soluble, and heavier than air in aqueous solutions were selected in the second-generation microbubbles. The liquid phase was an aqueous solution of polyvinyl alcohol (PVA) (Mw 13,000-23,000, 87-89% hydrolyzed, Sigma-Aldrich) at a concentration of 8 wt. % in 10 mM Phosphate-Buffered Saline (PBS) solution in Milli-Q water.

Due to the slow kinetics of dissolution of the polymer and in order to ensure complete solubilization, PVA was stirred in the aqueous solution at 80°C overnight before being used as the continuous phase. PVA acts as both a surfactant and cryoprotectant and PVA allows for the formation of a shell around the microbubbles; which is a barrier to reduce gas transport across the bubble interface and thereby increase their lifetimes from several tens of ms to several minutes, a cryoprotectant is added to avoid destruction of the microbubbles during the freeze-drying process (Mensink et al., How sugars protect proteins in the solid state and during drying (review): Mechanisms of stabilization in relation to stress conditions, Eur. J. Pharm. and Biopharm., 114 (2017) 288-295). The external fluid includes both cryoprotectant and surfactants, above CMC and below overlap concentration for the polymer surfactant and the cryoprotectant.

**EXAMPLE 3: Microfluidic microbubbles generation**

The liquid and gas flows were pressure-driven, $p_{liquid}$, and $p_{gas}$, using an MFCS-100 pressure controller system (Fluigent), at pressures from 1.2 to 3.5 bars. These, in turn, taking into account the geometrical features of the microfluidic chip as mentioned in **example 1**, enable to generate 5 μm diameter microbubbles (PDI <5%) with up to 10 kHz generation frequency with relatively high throughput (~$60 \times 10^4$ microbubbles/min) **(Figure 1)**. In situ microbubble generation was recorded using a high-speed camera (Photron SA3, Japan)-connected optical microscope (Zeiss Observer A1, USA) at 20x-40x magnification. The recordings were analyzed to obtain the size distribution and the generation rate of the microbubbles using MATLAB®.

**EXAMPLE 4: Freeze-drying of generated microbubbles: monolayer collection, pre-freeze, and lyophilization**

Bubbles generated according to **example 3** were transported along with the lateral outlet tubing. Then, the tip of the tube, from which around $10^4$ microbubbles exit the microfluidic chip in a drop of 0.1 $\mu$l, which can be characterized as a milky suspension, was moved on an $O_2$ plasma-treated hydrophilic glass slide (75 mm x 25 mm). Thus, the microbubbles were collected in a monolayer manner as individual bubble suspension spots on the slide (**Figure 2**). The repetition of this step may allow one to obtain one hundred spots on such surface, each of which consist of $10^4$ microbubbles with 5 $\mu$m in diameter.

Following the monolayer collection, the microbubbles were pre-frozen for 2h at -25°C, which is far below the glass transition temperature (Tg) of the used cryoprotectant PVA (~80°C). The frozen microbubbles were transferred to a freeze-dryer (FreeZone Labconco, USA) with a shelf temperature of -50°C for 4h.

As a result, monodisperse microbubbles, which are attached to the glass slide surface in the form of powder, referred to as freeze-dried cake, was obtained after the freeze-drying process (**Figure 5**).

**EXAMPLE 5: Traditional resuspension of the freeze-dried microbubbles**

The resuspension of 5 $\mu$m and 40 $\mu$m microbubbles with a PBS solution was performed in a traditional way, which comprises hydration of each spot with an injection of ~10 $\mu$l PBS solution, as shown in **Figure 6.** The size distribution analysis before and after freeze-drying showed that the size of microbubbles decreases less than 15% after freeze-drying. In both cases, monodispersity was kept below 6%.

Thus, the freeze-drying of monolayer-collected microbubbles in separate spots enabled the protection of the microbubbles during the process. The thickness of the shell of the dried microbubbles was estimated as 50 nm to 100 nm based on SEM images (**Figure 8**), which also revealed that its shell is smooth.

**EXAMPLE 6: Microfluidic resuspension of freeze-dried microbubbles**

Although the production rate of microfluidic microbubbles does not pose problems thanks to the parallelization, the monolayer constraint, from the collection to the resuspension, requires, in the present stage of the technology, the microbubbles to be placed on surfaces. Therefore, the packaging and thus, the resuspension of monodisperse freeze-dried microbubbles are different from that of traditional microbubbles. A typical glass-PDMS microfluidic chip which enables packaging and resuspension of around $10^6$ freeze-dried microbubbles that are present in the form of a monolayer on the glass slide was developed (**Figure 9**). The microfluidic resuspension system allows precise control of the bubble concentration, from 500 $\mu$l to 2 ml / $10^6$ microbubbles, typically with a PFC or $SF_6$ saturated PBS solution. At last, thanks to the permeability of PDMS, the complete packaging consists of the chip that is kept in a PFC or $SF_6$ ambient for carrying out hydration of microbubbles with the desired gas core. The chip was designed to provide a maximum surface area to cover a whole surface (75 mm x 25 mm) of a glass slide, which is supported by pillars to avoid collapsing the chip (**Figure 10**). The molds for the PDMS chip and the bonding of the chips to the glass slide that contains freeze-dried microbubbles were prepared as described earlier. This strategy is the product of a constraint, which is originated from the monolayer collection of microbubbles on a surface but enables controlled hydration of microbubbles and result in low-cost packaging. Based on the evidence in the scientific literature, the required injectable dose is diminished to which matches the amount that our freeze-dried technology can provide today, with only a few microfluidic chips, even without the need for massive parallelization, to meet the demand.

**EXAMPLE 7: Echogenicity of microbubbles for ultrasound contrast agent:**

The qualitative echogenicity of the freeze-dried PVA microbubbles with 5 $\mu$m in diameter was tested using an Aixplorer ultrasound system (V12). The probe used was the XC6-1 (192 transducers, piezo patch: 0.33 mm, probe radius: 60 mm). A sinusoidal burst centered on 3.21 MHz (bandwidth of 0.5 to 5 MHz) with a PRF =1000 Hz was sent. The freeze-dried microbubbles were transported to an institute located in Paris that has excellent expertise on acoustic imaging, LANGEVIN, then resuspended in a PBS solution, and ultrasound imaging experiments were carried out. A typical image is shown in **Figure 10.** Each bubble is clearly visible as white dots, one of which is marked by an arrow, on the echographic image. These experiments prove the echogenicity of PVA-shelled microbubbles according to the present invention, at a low mechanical index (MI=0.4) in response to a driving acoustic pulse produced by a conventional ultrasound transducer.

**EXAMPLE 8: Acoustic measurement of stationary bubbles in the chip:**

The stationary backscattered acoustic measurements of 5 $\mu$m PVA microbubbles were performed in a pillar-based microfluidic resuspension. Unlike the resuspension system, the PDMS microchannels were bonded to another blank-flat PDMS in order to avoid glass's strong acoustic reflectivity the acoustic impedance. The bubbles were generated as mentioned in example 3, but the outlet tubing (i.e. transfer tubing) was inserted to the inlet of the resuspension system. Then, the generated bubbles homogeneously distributed inside the chip and flowed until the outlet of the system.

When the bubbles started to come out from the chip, out of the outlet of the resuspension system, the chip was disconnected from the generation chip and the acoustic measurements were performed. A typical of $10^5$-$10^6$ bubbles was used for the measurements. For the reference measurement, the chip was filled with the deionized water and the acoustic measurement was performed.

A focused ultrasound transducer with a 38mm focal length and a central frequency of 2.25 MHz was used. The transducer was connected to an arbitrary wavefunction generator and a PicoScope 5242D for data acquisition. The transducer was used as an emitter and a receiver. Sinusoidal 1-cycle burst mode 5V signal is generated and used to excite bubbles. The used focus acoustic transducer was coupled with a 3D printed waveguide, which was fully filled with an acoustic gel that enabled propagation of a generated acoustic wave through the transducer to the first PDMS layer. Then, the wave travels along with the chip, as shown in **Figure 13A,** echoes were obtained from the first wall, microfluidic channel, and the back wall of the system. The echo came from the microfluidic channel that was analyzed with bubbles and without bubbles, with only water, thus the frequency spectrum of the bubbles was obtained and compared with that of reference measurement (**Figure 13B**).

The echo from the same bubble population was measured several times for 20 minutes. Each measurement consisted of 1000 measurements (1000 measurements take approximately 45 seconds) and each echo processed to FFT spectrum, then were averaged. Finally, the resulted resonance curves were Gaussian-fitted as shown in **Figure 12C.** The first three minutes (measurements A, B, C), we obtained 0.1% stability on the resonance frequency of the bubbles.

**Table 1** - results of the resonance frequency measurements according to Fig 12C

| Measurement | Time (minutes) | Resonance frequency (MHz) |
|---|---|---|
| A | 3 | 1.945 |
| B | 6 | 1.948 |
| C | 11 | 1.956 |
| D | 13 | 1.955 |
| E | 15 | 1.962 |
| F | 22 | 2.022 |

As shown in **Figure 14B,** there is no significant change in the size distribution of the bubbles after 3 min. After 20 minutes, bubbles polydispersity increased dramatically **(Figure 14D)** and resonance frequency stability was above 10%.

In conclusion, this result showed that our monodisperse bubbles are characterized with a narrow-band (around 15 %) resonance response with high stability (0.1 %), which enables to track the resonance frequency change, due to ambient pressure change, with 0.1 % (leading to sub-kPa pressure sensitivity), which is 10-fold better than the clinically required sensitivity ($\pm$ 2 kPa). Microbubbles available up to now suffer from achieving this sensitivity and performing reproducible measurements due to their broadband resonance response originated from their polydispersity nature.

**Claims**

**1.** Freeze-dried monodisperse microbubbles, wherein:

   • the microbubbles comprise a shell and a core,
   said shell in particular having a thickness of from 50 to 100 nm, as measured by scanning electron microscopy,
   • the microbubbles have a size inferior to 50 $\mu$m, in particular of from 200 nm to 50 $\mu$m, more in particular of from 1 to 50 $\mu$m, even more in particular of from 1 to 5 $\mu$m,
   • the microbubbles have a polydispersity index below 10%, preferably below 6%, more in particular below 5%,

wherein the microbubbles are physiologically inert,
wherein the microbubbles are adsorbed on a hydrophilic surface in the form of at least one monolayer,
in particular wherein the microbubbles are embedded in a film formed by the shell material.

2. Freeze-dried monodisperse microbubbles according to claim 1, wherein the shell of the microbubbles comprises, or consists of:

   • a surfactant, in particular chosen from:

   ▪ an amphiphilic polymer,
   ▪ water-soluble proteins, or
   ▪ phospholipids,
   or mixtures of said polymer, said water-soluble-proteins, and said phospholipids,
   and/or

   • a cryoprotectant,

wherein said amphiphilic polymer is in particular both the surfactant and the cryoprotectant,
and/or wherein the core of the microbubbles comprises or consists of a gas, preferably a hydrophobic gas.

3. Freeze-dried monodisperse microbubbles according to claim 2, wherein the amphiphilic polymer is a water-soluble amphiphilic polymer chosen from the group consisting of:

   polyvinyl alcohol (PVA), polyvinylpyrrolidone (PVP), poly(lactic-co-glycolic acid) (PLGA) and chitosan,
   or mixtures of said amphiphilic polymers,
   the amphiphilic polymer preferably being polyvinyl alcohol,
   wherein the water-soluble protein is serum albumin,
   wherein the phospholipid is chosen from the group consisting of:

   1,2-dipalmitoyl-sn-glycero-3 -phosphate (DPPA), 1,2-dipalmitoyl-sn-glycero-3-phosphocholine (DPPC), 1,2-dipalmitoyl-sn-glycero-3-phosphocholine (DSPC), 1,2-distearoyl-sn-glycero-3-phosphoethanolamine (DSPE),
   or mixtures of said phospholipids,
   the phospholipid preferably being DPPA;

   wherein the cryoprotectant is chosen from the group consisting of:

   • hydrophilic polymers, in particular hydrophilic polymers comprising hydroxyl groups, more in particular hydrophilic polymers chosen from the group consisting of polyvinyl alcohol (PVA), polyvinylpyrrolidone (PVP), poly(lactic-co-glycolic acid) (PLGA) and chitosan,
   • sugars,

   the cryoprotectant preferably being polyvinyl alcohol,
   and/or wherein the gas is chosen from:

   air, $SF_6$, or perfluorinated hydrocarbons, or mixtures of these gases,
   in particular perfluorohexane, or a mixture of perfluorohexane and $SF_6$.

4. Freeze-dried monodisperse microbubbles according to anyone of claims 1 to 3, wherein the hydrophilic surface is a glass surface, in particular a sodium silicate, or boro silicate glass surface,
and/or wherein said hydrophilic surface, is flat or curbed, preferably flat, in particular a flat glass slide.

5. Freeze-dried monodisperse microbubbles according to any of claims 1 to 4, wherein the microbubbles are adsorbed on a hydrophilic surface in the form of a single monolayer, said monolayer being continuous, uninterrupted by regions that are devoid of microbubbles,
or wherein the microbubbles are adsorbed on a hydrophilic surface in the form of more than one monolayers, in particular of from 2 to 1000 monolayers, preferably about 100 monolayers, said more than one monolayers being non-contiguous with respect to each other,

in particular wherein the distance between a first monolayer and a second monolayer, adjacent and non-contiguous to the first monolayer, is substantially identical to the distance between any one of two adjacent non-contiguous monolayers on the hydrophilic surface.

6. Process for preparing freeze-dried microbubbles, wherein the microbubbles:

   • comprise a shell and a core,
   said shell in particular having a thickness of from 50 to 100 nm, as measured by scanning electron microscopy,
   • have a size inferior to 50 $\mu$m, in particular of from 200 nm to 50 $\mu$m, more in particular of from 1 to 50 $\mu$m, even more in particular of from 1 to 5 $\mu$m,
   • the microbubbles have a polydispersity index below 10%, preferably below 6%, more in particular below 5%,

   wherein the microbubbles are physiologically inert,
   wherein the microbubbles are adsorbed on a hydrophilic surface in the form of at least one monolayer,
   in particular wherein the microbubbles are embedded in a film formed by the shell material,
   said process comprising a step of:

   • freeze-drying monodisperse microbubbles adsorbed on a hydrophilic surface in the form of at least one monolayer, to obtain freeze-dried monodisperse microbubbles adsorbed on a hydrophilic surface in the form of at least one monolayer,
   said step of freeze-drying being in particular carried out for a time of approximately 4 hours,

   and optionally, before the step of freeze drying, a step of:

   • freezing the monodisperse microbubbles adsorbed on a hydrophilic surface in the form of at least one monolayer, to obtain frozen monodisperse microbubbles adsorbed on a hydrophilic surface in the form of at least one monolayer,

   said step of freezing being in particular carried out for a time of approximately 2 hours,
   said steps of freeze-drying and/or said step of freezing being in particular carried out at a temperature below the glass transition temperature of the cryoprotectant.

7. Process according to claim 6, wherein the process further comprises:

   before the step of freeze-drying or before the optional step of freezing, a step of

   • collecting monodisperse microbubbles on a hydrophilic surface in the form of at least one monolayer, to obtain monodisperse microbubbles adsorbed on a hydrophilic surface in the form of at least one monolayer,

   and optionally, before the step of collecting, a step of

   • forming monodisperse microbubbles using a microfluidic chip, in particular a flow-focusing chip,
   said step of forming monodisperse microbubbles comprising a step of microfluidic bubbling of a gas phase, through a liquid phase, comprising an aqueous solution of a surfactant and/or a cryoprotectant,
   to obtain monodisperse microbubbles,
   said gas phase forming the core, and said liquid phase forming the shell.

8. Process according to claim 7, wherein the step of collecting the monodisperse microbubbles comprises:

   • contacting said hydrophilic surface with the tip of a tube through which said microbubbles exit from the microfluidic chip, to obtain a single monolayer of microbubbles,
   said step of contacting being in particular carried out for a period of 1 to 3 seconds,

   or, wherein the step of collecting the monodisperse microbubbles comprises:

   • contacting said hydrophilic surface with the tip of the tube through which said microbubbles exit from the microfluidic chip, to obtain a first monolayer of microbubbles,
   said step of contacting being in particular carried out for a period of 1 to 3 seconds,

• shifting the tip of the tube or the hydrophilic surface to a position enabling deposition of a second monolayer of microbubbles adjacent and non-contiguous to the first monolayer of microbubbles, during a further step of contacting said hydrophilic surface with said tip,
to obtain a second monolayer of microbubbles, adjacent to the first monolayer of microbubbles, said first and second monolayer being non-contiguous,
• optionally repeating the steps of shifting and contacting n times, to obtain (n+1) monolayers, n being an integer greater than 1.

9. Process according to any of claims 6 to 8, wherein the liquid phase comprises:

    • a surfactant, in particular chosen from:

        ▪ an amphiphilic polymer,
        ▪ water-soluble proteins, or
        ▪ phospholipids,
      or mixtures of said polymer, said water-soluble-proteins, and said phospholipids,
      and/or

    • a cryoprotectant,

wherein said amphiphilic polymer is in particular both the surfactant and the cryoprotectant, preferably polyvinyl alcohol.
and/or wherein the gas phase comprises or consists of a gas, preferably a hydrophobic gas.

10. Process according to claim 9, wherein the amphiphilic polymer is a water-soluble amphiphilic polymer chosen from the group consisting of:

    polyvinyl alcohol (PVA), polyvinylpyrrolidone (PVP), poly(lactic-co-glycolic acid) (PLGA) and chitosan,
    or mixtures of said amphiphilic polymers,
    the amphiphilic polymer preferably being polyvinyl alcohol,
    wherein the water-soluble protein is serum albumin,
    wherein the phospholipid is chosen from the group consisting of:

        1,2-dipalmitoyl-sn-glycero-3 -phosphate (DPPA), 1,2-dipalmitoyl-sn-glycero-3 - phosphocholine (DPPC), 1,2-dipalmitoyl-sn-glycero-3-phosphocholine (DSPC), 1,2-distearoyl-sn-glycero-3-phosphoethanolamine (DSPE),
        or mixtures of said phospholipids,
        the phospholipid preferably being DPPA;

    wherein the cryoprotectant is chosen from the group consisting of:

        • hydrophilic polymers, in particular hydrophilic polymers comprising hydroxyl groups, more in particular hydrophilic polymers chosen from the group consisting of polyvinyl alcohol (PVA), polyvinylpyrrolidone (PVP), poly(lactic-co-glycolic acid) (PLGA) and chitosan,
        • sugars,

    the cryoprotectant preferably being polyvinyl alcohol,
    and/or wherein the gas is chosen from:

        air, $SF_6$, or perfluorinated hydrocarbons, or mixtures of these gases,
        in particular perfluorohexane, or a mixture of perfluorohexane and $SF_6$.

11. Freeze-dried monodisperse microbubbles adsorbed on a hydrophilic surface in the form of at least one monolayer, such as obtained by the process according to anyone of claims 6 to 10.

12. Process for preparing an aqueous suspension of monodisperse microbubbles, wherein the process comprises:

    • a step of resuspending freeze-dried monodisperse microbubbles adsorbed on a hydrophilic surface according to any of claims 1 to 5, to obtain an aqueous suspension of monodisperse microbubbles,
wherein the microbubbles are physiologically inert,

said step of resuspending comprising:
contacting a freeze-dried monolayer of monodisperse microbubbles as previously described with an aqueous medium, preferably a buffer, more preferably phosphate buffered saline,
said step of resuspending being in preferably carried out in a microfluidic chip, in particular in a microfluidic chip comprising pillar based microchannels, or comprising slit microchannels.
said aqueous medium being in particular saturated with a gas, in particular a gas chosen from $SF_6$, air, or perfluorinated hydrocarbons, in particular perfluorohexane, or a mixture of perfluorohexane and $SF_6$,
said gas, or mixture of gases, being preferably the same as the gas, or mixture of gases, comprised in, or constituting the core of said microbubbles.

13. An aqueous suspension of freeze-dried monodisperse microbubbles, such as obtained by the process of claim 12, or an aqueous suspension of monodisperse microbubbles, wherein:

• the microbubbles comprise a shell and a core,
said shell in particular having a thickness of from 50 to 100 nm, as measured by scanning electron microscopy,
• the microbubbles have a size inferior to 50 $\mu$m, in particular of from 200 nm to 50 $\mu$m, more in particular of from 1 to 50 $\mu$m, even more in particular of from 1 to 5 $\mu$m,
• the microbubbles have a polydispersity index below 10%, preferably below 6%, more in particular below 5%,

wherein the microbubbles are physiologically inert,
wherein the monodisperse microbubbles are substantially devoid of organic solvents, in particular devoid class 1 and/or class 2 solvents, in particular chloroform or dichloromethane,
wherein the aqueous suspension comprises an aqueous medium in the form of a buffer, preferably phosphate buffered saline,
wherein the shell preferably comprises polyvinyl alcohol, and wherein the core preferably comprises a hydrophobic gas.

14. Use of the aqueous suspension of freeze-dried monodisperse microbubbles according to claim 13, or of the freeze-dried monodisperse microbubbles according to any of claims 1 to 5 or 11:

• as an ultrasound contrast agent for molecular imaging,
• as an ambient pressure sensor in non-invasive blood-pressure measurements,
• as ultrasound agents for sonoporation,
• as acoustic biosensors,

in particular as an ultrasound contrast agent.

15. a microfluidic chip comprising:

• inside the chip, a hydrophobic surface, preferably a glass slide, on which monodisperse microbubbles are absorbed in the form of at least one monolayer, according to any of claims 1 to 5 or 11,
• one or more inlets for supplying the aqueous medium
• one or more outlets for collecting the resuspended microbubbles.

**Figure 1**

**Figure 2**

Figure 3

Figure 4

Figure 5

Figure 6

Figure 7

Figure 8

Figure 9

Figure 10

Figure 11

Figure 12

Figure 13

Figure 14

**Figure 15**

Europäisches
Patentamt

European
Patent Office

Office européen
des brevets

# EUROPEAN SEARCH REPORT

Application Number

EP 20 20 1537

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/017524 A1 (UNIV PENNSYLVANIA [US]; LEE DAEYEON [US] ET AL.) 10 February 2011 (2011-02-10) * page 11, lines 35-37; figure 3; examples 2-4 * | 1-15 | INV. B01J13/04 A61K9/127 A61K9/50 A61K47/69 A61K49/22 |
| X | FESHITAN J A ET AL: "Microbubble size isolation by differential centrifugation", JOURNAL OF COLLOID AND INTERFACE SCIENCE, ACADEMIC PRESS,INC, US, vol. 329, no. 2, 15 January 2009 (2009-01-15), pages 316-324, XP025677572, ISSN: 0021-9797, DOI: 10.1016/J.JCIS.2008.09.066 [retrieved on 2008-10-01] * point 2.2 beginning on page 317, point 2.8 beginning on page 318 and table 1 of page 321 * | 13 | |
| A | US 2008/182056 A1 (BAKKER ERIC [US] ET AL) 31 July 2008 (2008-07-31) * claim 1; example 1 * | 1-15 | TECHNICAL FIELDS SEARCHED (IPC) B01J A61K |
| A | US 2018/369231 A1 (PARK YOUNG HWAN [KR] ET AL) 27 December 2018 (2018-12-27) * example 5; table 5 * | 1-15 | |
| A | CHEN CHUANPIN ET AL: "Production of monodispersed micron-sized bubbles at high rates in a microfluidic device", APPLIED PHYSICS LETTERS, A I P PUBLISHING LLC, US, vol. 95, no. 14, 5 October 2009 (2009-10-05), pages 144101-144101, XP012126017, ISSN: 0003-6951, DOI: 10.1063/1.3242019 * figure 2 * | 1-15 | |

The present search report has been drawn up for all claims

| Place of search | Date of completion of the search | Examiner |
|---|---|---|
| The Hague | 28 February 2021 | Mc Donnell, Shane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 20 20 1537

This annex lists the patent family members relating to the patent documents cited in  the above-mentioned European search report.
The members are as contained in the European Patent Office EDP file on
The European Patent Office is in no way liable for these particulars which are merely given for the purpose of information.

28-02-2021

| Patent document cited in search report | | Publication date | Patent family member(s) | | Publication date |
|---|---|---|---|---|---|
| WO 2011017524 | A1 | 10-02-2011 | US | 2012328529 A1 | 27-12-2012 |
| | | | WO | 2011017524 A1 | 10-02-2011 |
| US 2008182056 | A1 | 31-07-2008 | US | 2008182056 A1 | 31-07-2008 |
| | | | WO | 2008095007 A1 | 07-08-2008 |
| | | | WO | 2008124202 A2 | 16-10-2008 |
| US 2018369231 | A1 | 27-12-2018 | AU | 2018289188 A1 | 05-12-2019 |
| | | | BR | 112019024744 A2 | 16-06-2020 |
| | | | CA | 3064564 A1 | 27-12-2018 |
| | | | CN | 110769813 A | 07-02-2020 |
| | | | EP | 3641730 A1 | 29-04-2020 |
| | | | JP | 2020524712 A | 20-08-2020 |
| | | | KR | 20190000325 A | 02-01-2019 |
| | | | US | 2018369231 A1 | 27-12-2018 |
| | | | WO | 2018236190 A1 | 27-12-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SEGERS.** *RSC Nanoscience and Nanotechnology,* 2015, 81-101
- **S. PODELL.** Physical and biochemical stability of Optison®, an injectable ultrasound contrast agent. *Biotechnol. Appl. Biochem.,* 1999, vol. 30, 213-223
- **PATRICK TABELING.** Introduction to Microfluidics. Oxford University Press, 2005
- **J. COOPER MCDONALD et al.** Fabrication of microfluidic systems in poly(dimethylsiloxane. *Electrophoresis,* 2000, vol. 21, 27-40
- **MALLOGGI et al.** Monodisperse Colloids Synthesized with Nanofluidic Technology. *Langmuir,* 2010, vol. 26, 2369-2373
- **MENSINK et al.** How sugars protect proteins in the solid state and during drying (review): Mechanisms of stabilization in relation to stress conditions. *Eur. J. Pharm. and Biopharm.,* 2017, vol. 114, 288-295